(19) Europäisches Patentamt
European Patent Office
Office européen des brevets

(11) **EP 3 687 627 B1**

(12) **EUROPÄISCHE PATENTSCHRIFT**

(45) Veröffentlichungstag und Bekanntmachung des Hinweises auf die Patenterteilung:
**15.12.2021 Patentblatt 2021/50**

(21) Anmeldenummer: **18814769.8**

(22) Anmeldetag: **27.09.2018**

(51) Int Cl.:
***A61N 5/10*** *(2006.01)*

(86) Internationale Anmeldenummer:
**PCT/DE2018/000275**

(87) Internationale Veröffentlichungsnummer:
**WO 2019/063035 (04.04.2019 Gazette 2019/14)**

(54) **VERFAHREN ZUR EPID-BASIERTEN ÜBERPRÜFUNG, KORREKTUR UND MINIMIERUNG DES ISOZENTRUMS EINES STRAHLENTHERAPIEGERÄTS**

METHOD FOR EPID-BASED VERIFICATION, CORRECTION AND MINIMISATION OF THE ISOCENTRE OF A RADIOTHERAPY DEVICE

PROCÉDÉ DE VÉRIFICATION, DE CORRECTION ET DE MINIMISATION À BASE D'ÉPID DE L'ISOCENTRE D'UN DISPOSITIF DE THÉRAPIE PAR RAYONNEMENT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **27.09.2017 DE 102017009040**

(43) Veröffentlichungstag der Anmeldung:
**05.08.2020 Patentblatt 2020/32**

(73) Patentinhaber: **Städtisches Klinikum Dessau 06847 Dessau-Roßlau (DE)**

(72) Erfinder: **WÖSLE, Markus 06844 Dessau-Roßlau (DE)**

(74) Vertreter: **Dinter, Tilo Dinter, Kreißig & Partner Gottschedstr. 12 04109 Leipzig (DE)**

(56) Entgegenhaltungen:
**US-A1- 2015 360 056**

- **Daniel E Hyer ET AL: "Development and implementation of an EPID-based method for localizing isocenter", Journal of applied clinical medical physics / American College of Medical Physics, 1. Januar 2012 (2012-01-01), Seite 3965, XP055168550, United States DOI: 10.1120/jacmp.v13i6.3965 Gefunden im Internet: URL:http://www.ncbi.nlm.nih.gov/pubmed/231 49787**
- **PEJMAN ROWSHANFARZAD ET AL: "Isocenter verification for linac-based stereotactic radiation therapy: review of principles and techniques | Rowshanfarzad | Journal of Applied Clinical Medical Physics", JOURNAL OF APPLIED CLINICAL MEDICAL PHYSICS, Bd. 12, Nr. 4, 15. November 2011 (2011-11-15), XP055168728,**

**Beschreibung**

Die Erfindung betrifft ein Verfahren zur Überprüfung, Korrektur und Minimierung des Isozentrums eines Strahlentherapiegeräts, welches zumindest einen um mindestens eine Tischachse drehbaren Patientenlagerungstisch, einen um eine Tragarmachse drehbaren Tragarm, einen an dem Tragarm angeordneten Strahlerkopf zur Erzeugung eines Therapiestrahls, einen drehbaren Collimator, eine Einrichtung zum Projizieren des radiologischen Isozentrums und ein tragarmfestes digitales Aufnahmesystem (EPID, englisch: Electronic Portal Imaging Device) zum Erfassen von Dosisbildern mittels des Therapiestrahls umfasst.

Bei der Behandlung von Tumorerkrankungen mittels ionisierender Strahlung besteht eine wesentliche Herausforderung darin, dem Krankheitsherd die therapeutische Energiedosis zu verabreichen und das benachbarte gesunde Gewebe bestmöglich zu schonen. Erreicht wird dies durch mechanisch und dosimetrisch präzise Strahlentherapiegeräte, wie beispielsweise Elektronen-Linearbeschleuniger, Gamma-Knifes, Cyber-Knifes, Protonen- und Schwerionen-Beschleunigeranlagen in Verbindung mit an Maschinen- und Patientenlagerungstoleranzen angepassten Sicherheitssäumen bei der Zielvolumendefinition. Ein zentrales Merkmal jedes Strahlentherapiegeräts ist die räumliche Abweichung des Zentralstrahls vom idealen punktförmigen Ort, der Isozentrum oder ideales Isozentrum genannt wird. Bei einem idealen Strahlentherapiegerät, bei dem keine mechanischen Toleranzen auftreten, schneiden sich alle winkelabhängigen Zentralstrahlen in diesem Isozentrum. Medizinische Elektronen-Linearbeschleuniger, die in nahezu jeder Strahlentherapie-Institution zur Tumortherapie eingesetzt werden, besitzen mindestens drei rotatorische Freiheitsgrade. Hierzu gehören

- der Tragarmwinkel mit einem üblichen Wertebereich von -180° bis +180°,
- der Collimatorwinkel mit einem üblichen Wertebereich von -175° bis + 175° und
- der isozentrische Winkel des Patientenlagerungstisches mit einem üblichen Wertebereich von -95° bis +95°

Für ein reales Strahlentherapiegerät ist das Isozentrum kein Punkt, sondern ein Isozentroid mit räumlicher Ausdehnung (im Folgenden auch räumliches Isozentrum genannt). Der Begriff Zentroid ist von Ellipsoid abgeleitet, welches ein durch Drehung einer Ellipse um eine ihrer Achsen entstehender Körper ist. Die Zentralstrahlen von sämtlichen Kombinationen der oben genannten Winkel schneiden oder berühren das Isozentroid. Zur Bestimmung des globalen Isozentroids eines Strahlentherapiegeräts sind zunächst die jeweilige Größe und Lage der einzelnen Isozentroide in Abhängigkeit jedes einzelnen Winkelfreiheitsgrades zu ermitteln. Diese Messung und Verifikation der Isozentroide ist auch als Winston-Lutz-Test oder Winston-Lutz-Methode bekannt.

Beim Winston-Lutz-Test wird zunächst ein röntgendichter beziehungsweise strahlungsdichter Messkörper, der auch als Winston-Lutz-Pointer bezeichnet wird und mit dem Patientenlagerungstisch eines Strahlentherapiegeräts starr gekoppelt ist, im radiologischen Isozentrum eines Strahlentherapiegeräts positioniert. Dieses Isozentrum wird mit Hilfe von raumfesten, sichtbaren Linienlasern angezeigt beziehungsweise auf den Winston-Lutz-Pointer projiziert. Im Anschluss an die Positionierung des Messkörpers wird mit einem quadratischen oder runden Bestrahlungsfeld, das durch die Blockblenden eines Collimators, die Lamellen eines Multi-Leaf-Collimators (MLC) beziehungsweise durch einen zusätzlich am Strahlerkopf montierten Rundcollimator begrenzt wird, jeweils ein ebenes Dosisbild bei diversen Tragarmwinkeln, Collimatorwinkeln und Tischwinkeln mittels eines digitalen Aufnahmesystems (EPID) angefertigt. Anschließend wird eine digitale Bildverarbeitung der angefertigten Dosisbilder durchgeführt, wobei verschiedene Algorithmen eingesetzt werden, um die räumlich Lage des Zentralstrahls relativ zum Messkörper bestimmen zu können. Die bekannten Verfahren und Algorithmen beruhen beispielsweise auf digitalen Hochpassfiltern, zweidimensionalen Operatoren zur Kantenverstärkung und Kantenfindung (wie beispielsweise Sobel-Operatoren, Canny-Filter), der Linienextraktion (Hough-Transformation), der Berechnung des Schwerpunktes (Centre of Mass, Signal Intensity Weighting), der Faltungsmethode (Convolution), der Objektsegmentierung (Segmentation, Contouring) sowie auf dem Schwellwertverfahren (Thresholding). In der Übersichtsarbeit "Isocenter verification for linac-based stereotactic radiation therapy: review of principles and techniques" Rowshanfarzad, P., Sabet, M., O'Connor, D. J., Greer, P. B. (Journal of Applied Clinical Medical Physics, Volume 12, No. 4, 185-195, 2011) werden die oben genannten Methoden erläutert sowie deren Vor- und Nachteile diskutiert. Die wesentlichen Nachteile der EPID-basierten Methoden zur Isozentrumsverifikation bestehen gegenüber filmbasierten Methoden in einem vergleichsweise geringeren Auflösungsvermögen, wodurch eine exakte Verifikation des radiologischen Isozentrums erschwert ist. In einer aktuellen Veröffentlichung "A study of Winston-Lutz test on two different electronic portal imaging devices and with low energy imaging" Ravindran, P.B. (Australasian Physical and Engineering Sciences in Medicine, Volume 39, Issue 3, 677-685, 2016) wird der Winston-Lutz-Pointer real mit der minimalen Schrittweite 0.25 mm verschoben. Wie daraus weiter hervorgeht wird mittels morphologischer Operationen ein maximaler Fehler der Zentralstrahlabstände vom Winston-Lutz-Pointer von $0.04 \pm 0.02$ mm ermittelt. Aus der Publikation "A robust Hough transform algorithm for determining the radiation centers of circular and rectangular fields with subpixel accuracy" Du, W., Yang, J. (Phys. Med. Biol., 54(3), 555-567, 2009) ist es bekannt, mittels Hough-Transformation in rechnerisch variierten Dosisbildern eine theoretische Genauigkeit von $0.02 \pm 0.01$ mm zu erreichen, wobei Maschinentoleranzen nicht berücksichtigt werden.

Weitere Probleme der bekannten Methoden bestehen in der Unmöglichkeit einer Messdurchführung bei bestimmten Tragarmwinkel-/Tischwinkelkombinationen und in einem ungünstigen Signal-/Rauschverhältnis.

Bei EPID-basierten Methoden besteht weiterhin das Problem, dass Auswertungsverfahren, wie die Hough-Transformation für MLC-geformte Bestrahlungsfelder ungeeignet sind, da die Lamellen infolge endlicher Positionierungsgenauigkeit und Durchlassstrahlung zwischen den Lamellen mit ihren Enden keine geraden Feldgrenzen bilden können.

Von Nachteil ist außerdem, dass die Positionierungsungenauigkeiten des Aufnahmesystems bei allen EPID-basierten Methoden - insbesondere eine Verletzung der Orthogonalität zum Zentralstrahl und ein nicht exakter Abstand zum Isozentrum - das Ergebnis beeinflussen. Weitere Mängel bei den bekannten EPID-basierten Methoden sind eine fehlende Darstellung des globalen Isozentroids, worin die Zentralstrahlabweichung vom idealen Isozentrum für eine beliebige Kombination der drei Winkel für den Tragarm, Collimator und Patientenlagerungstisch ersichtlich ist, eine fehlende mathematische Beschreibung des globalen Isozentroids zu seiner Minimierung mittels Optimierung von Parametern des Strahlentherapiegeräts sowie fehlende Untersuchungen des Einflusses aller Messbedingungen auf das Ergebnis.

Die der vorliegenden Erfindung zugrundeliegende Aufgabe besteht nunmehr darin, ein EPID-basiertes Verfahren zur Überprüfung und Korrektur des Isozentrums eines Strahlentherapiegeräts vorzuschlagen, mit dem die aus dem Stand der Technik bekannten Nachteile vermieden oder verringert werden können.

Erfindungsgemäß wird die Aufgabe mit einem Verfahren mit den Merkmalen des Anspruchs 1 gelöst. Vorteilhafte Ausgestaltungen und Weiterbildungen der Erfindung können mit in untergeordneten Ansprüchen bezeichneten Merkmalen realisiert werden.

Gemäß der Aufgabenstellung wird erfindungsgemäß ein Verfahren zur EPID-basierten Überprüfung, Korrektur und Minimierung des Isozentrums eines Strahlentherapiegeräts, welches mindestens einen um eine Tischachse drehbaren Patientenlagerungstisch, einen um eine Tragarmachse drehbaren Tragarm, einen an dem Tragarm angeordneten Strahlerkopf zur Erzeugung eines Therapiestrahls, einen drehbaren Collimator, eine Einrichtung zum Projizieren des radiologischen Isozentrums und ein digitales Aufnahmesystem (EPID) zum Erfassen von Dosisbildern mittels des Therapiestrahls umfasst, vorgeschlagen, bei dem wie folgt vorgegangen wird:

a) Ein Messkörper wird mittels der Projektionseinrichtung an der Projektionsposition im momentanen radiologischen Isozentrum des Strahlentherapiegeräts positioniert,

b) ein mit dem Collimator begrenztes Bestrahlungsfeld wird für mindestens eine vorgegebene Winkeleinstellung des Tragarms, des Patientenlagerungstischs und des Collimators appliziert, und dabei wird

c) mit dem EPID mindestens ein gemeinsames Dosisbild des Messkörpers und des Bestrahlungsfeldes aufgenommen,

d) anhand des aufgenommenen Dosisbildes wird jeweils ein Dosisprofil für jede Richtung innerhalb eines EPID-Koordinatensystems des EPID erstellt und

e) im Verlauf des Dosisprofils wird an beiden zu erwartenden Körpergrenzen des Messkörpers in X-Richtung des EIPD-Koordinatensystems und an beiden zu erwartenden Körpergrenzen des Messkörpers in Y-Richtung des EPID-Koordinatensystems zwischen einem lokalen Dosisminimum und einem lokalen Dosismaximum und zwischen einem lokalen Dosismaximum und einem lokalen Dosisminium jeweils ein Wendepunkt ermittelt und

f) die ermittelten Positionen der Wendepunkte werden den Körpergrenzen des Messkörpers in X-Richtung und in Y-Richtung zugeordnet,

g) anhand der zugeordneten Körpergrenzen des Messkörpers wird im Dosisbild eine Lage des Mittelpunkts des Messkörpers relativ zu dem EPID-Zentrum ermittelt, wobei die Schritte d) bis g) analog für die Feldgrenzen und den Feldmittelpunkt des Bestrahlungsfeldes durchgeführt werden, das heißt, es wird anhand des aufgenommenen Dosisbildes ein Dosisprofil erstellt und im Verlauf des Dosisprofils wird an beiden zu erwartenden Feldgrenzen des Bestrahlungsfeldes in X-Richtung des EIPD-Koordinatensystems und an beiden zu erwartenden Feldgrenzen des Bestrahlungsfeldes in Y-Richtung des EPID-Koordinatensystems zwischen einem lokalen Dosisminimum und einem lokalen Dosismaximum und zwischen einem lokalen Dosismaximum und einem lokalen Dosisminium jeweils ein Wendepunkt ermittelt und die ermittelten Positionen der Wendepunkte der Feldgrenzen des Bestrahlungsfeldes in X-Richtung und in Y-Richtung zugeordnet und anhand der zugeordneten Feldgrenzen des Bestrahlungsfeldes im Dosisbild eine Lage des Feldmittelpunkts des Bestrahlungsfeldes relativ zu dem EPID-Zentrum ermittelt,

h) aus einer Lageabweichung des Mittelpunktes des Messkörpers von dem EPID-Zentrum und aus einer Lageabweichung des Mittelpunktes des Bestrahlungsfeldes vom EPID-Zentrum wird ein Differenzvektor bestimmt, und

i) die Vektorkomponenten des Differenzvektors werden zur Korrektur des momentanen radiologischen Isozentrums eingesetzt.

In Schritt d) kann zur Bestimmung von absoluten Abständen ein Einheitenwechsel von Pixel zu mm vorgenommen werden. Der Einheitenwechsel stellt einen Übergang vom digitalen zum analogen Dosisprofil dar, sodass eine Weiterverarbeitung der Geometriedaten in einem Algorithmus gewährleistet werden kann.

Das für den Messaufbau und für die Durchführung des erfindungsgemäßen Verfahrens wesentliche Koordinatensystem ist das Inertialsystem (raumfestes Strahlentherapiegerät-Koordinatensystem), in dem die Isozentrumsabweichungen (Zentralstrahlabweichungen vom idealen Isozentrum) angegeben werden. Das Inertialsystem hat das ideale Isozentrum ISO (Mittelpunkt des Messkörpers beziehungsweise der Wolframkugel des Winston-Lutz-Pointers) als Ursprung. Da das für die Schritte c) bis h) verwendete Koordinatensystem des Aufnahmegerätes sowie das Collimator-Koordinatensystem beweglich sind, werden Koordinatentransformationen benötigt. Bei der Variation des Tragarmwinkels (G) wird der jeweilige Differenzvektor mittels der linken Matrix in der weiter unten aufgeführten Gleichung (5) in das Intertialsystem abgebildet, wobei $\beta$ = G ist. Bei der Variation des Collimatorwinkels (C) werden die Dosisbilder vor der Analyse mittels der rechten Matrix in Gleichung (5) in das EPID-Koordinatensystem abgebildet, wobei $\gamma$ = -C ist; nach der Analyse werden die Vektoren mit derselben Matrix, wobei $\gamma$ = C ist, in das Inertialsystem abgebildet. Bei der Variation des Patientenlagerungstischwinkels (T) mit G $\neq$ 0° ist wieder die linke Matrix in Gleichung (5) mit $\beta$ = G zur Koordinatentransformation notwendig. Da infolge von Maschinentoleranzen - insbesondere von Positionierungsfehlern des Aufnahmesystems - Fehler im Ergebnis auftreten, werden alle Differenzvektoren vor der Abbildung ins Inertialsystem gemäß der weiter unten angeführten Gleichung (2) vom realen in das ideale Koordinatensystem abgebildet. Relevant ist weiterhin das EPID-Koordinatensystem, welches tragarmfest positioniert ist und seinen Ursprung im zentralen Detektorelement des EPID hat. Vorzugsweise erfolgt die Projektion des momentanen radiologischen Isozentrums mittels fünf raumfester Linienlaser. Mit Fadenkreuzen, die in Form von farblich hervorgehobenen Einkerbungen auf der Oberfläche des Messkörpers ausgeführt sein können, kann der Messkörper im projizierten Isozentrum des Strahlentherapiegeräts exakt positioniert werden.

Nach einer vorteilhaften Weiterbildung des erfindungsgemäßen Verfahrens können die Verfahrensschritte b) bis h) für die Winkelfreiheitsgrade des Patientenlagerungstischs, des Tragarms und des Collimators mit einem Inkrement von höchstens 30° durchgeführt werden. Zweckmäßigerweise können dabei auch solche Winkeleinstellungen gewählt werden, die für eine nachfolgende Behandlung eines Patienten relevant sind.

Weiter kann es vorgesehen sein, dass aus Dosisbildern, die von verschiedenen Winkelpositionen des Patientenlagerungstischs, des Tragarms und des Collimators stammen, ermittelbare Differenzvektoren zur Bestimmung der Größe und Lage der räumlichen Isozentren eingesetzt werden, wobei die Vektorkomponenten der Ortsvektoren der räumlichen Isozentren zur Korrektur des radiologischen Isozentrums eingesetzt werden.

Das erfindungsgemäße Verfahren kann vorzugsweise automatisch durchgeführt werden, wobei eine entsprechende Software eingesetzt wird. Eine automatische Durchführung der Schritte d) bis i) und insbesondere eine Auswertung von Dosisprofilen zum Auffinden von Wendepunkten sowie die Durchführung von Koordinatentransformationen können mittels Routinen, beispielsweise im Softwarepaket MATLAB®, erfolgen.

Vorzugsweise kann in Schritt e) das Dosisprofil des Dosisbilds in X-Richtung und in Y-Richtung auf Wendepunkte zwischen einem Dosisminimum und einem Dosismaximum sowie im weiteren Verlauf zwischen einem Dosismaximum und einem Dosisminimum untersucht werden, um sämtliche Körpergrenzen des Messkörpers und Grenzen des Bestrahlungsfeldes zu ermitteln. Idealerweise handelt es sich bei dem Messkörper um eine Wolframkugel mit einem Durchmesser zwischen 5 mm und 10 mm. Dabei ist anzumerken, dass die bei der Fehlermöglichkeitsanalyse und Fehlereinflussanalyse gefundenen Parameterwerte für geeignete Messbedingungen und Analysebedingungen vorzugsweise für einen Kugeldurchmesser von 5 mm gelten. Das erfindungsgemäße Verfahren soll zur Durchführung jedoch nicht auf einen kugelförmigen Messkörper beschränkt sein. Es sind daher auch andere Körperformen des Messkörpers denkbar.

Nach einer Konzeption der Erfindung entspricht die zweidimensionale Abbildung des Durchmessers eines kugelförmigen Messkörpers und der Feldbreite eines Bestrahlungsfeldes im Dosisbild dem Abstand zwischen zwei Wendepunkten im Dosisprofil parallel zur X-Richtung des collimatorfesten Blendenkoordinatensystems. Dabei können als Wendepunkte diejenigen Punkte angesehen werden, die genau 50% der Energiedosis des Feldzentrums im Dosisbild aufweisen. Entsprechend können in Y-Richtung diejenigen Punkte als Wendepunkte angesehen werden, die genau 50% der Energiedosis des Feldzentrums im Dosisbild aufweisen. Die dabei ermittelten Wendepunkte werden den Feldgrenzen des Bestrahlungsfeldes zugeordnet. Da sich im Bereich des zweidimensionalen Feldzentrums das lokale Dosisminimum hinter dem Messkörper befindet, kann als Ersatz für die 100%-Dosis das kleinste der lokalen Dosismaxima der Dosisprofile in X-Richtung und Y-Richtung verwendet werden. Die Ermittlung der Körpergrenzen des Messkörpers erfolgt analog. Die Energiedosis zur Definition der Grenzen des kugelförmigen Messkörpers ist das arithmetische Mittel aus der 100%-Ersatzdosis und dem lokalen Dosisminimum hinter der Kugel, also eine 50%-Ersatzdosis. Die Dosismaxima befinden sich zwischen dem kugelförmigen Messkörper und den Feldrändern, die durch die Collimatoreinstellung definiert werden. Gemäß den vorstehenden Ausführungen kann es vorgesehen sein, dass der/die Wendepunkt/e im Bereich eines 50%-Dosispunktes zwischen einem Dosisminimum und einem Dosismaximum und/oder zwischen einem Dosismaximum und einem Dosisminium des Dosisprofils ermittelt wird/werden. Die Definition der Feldgröße und der Zentralstrahllage mit Hilfe der 50%-Isodose ist in der Norm IEC 60976 beschrieben.

Die zur Bildebene senkrechten Messebenen, die die entsprechenden Dosisprofile zur Definition der Feldgröße, des Zentralstrahls sowie der Kugelgröße und des Kugelmittelpunktes ausschneiden, können mit Hilfe einer Software unter Anwendung eines Algorithmus automatisch bestimmt werden. Ausgehend vom lokalen Dosisminimum im Dosisbild des kugelförmigen Messkörpers, der ungefähr im Bildzentrum liegt, können in einem ersten Iterationsschritt zwei orthogonale Messebenen definiert werden. Die dabei bestimmten Dosisprofile werden bezüglich Feldgröße, Zentralstrahllage, Kugelgröße und Kugelmittelpunkt analysiert. Im zweiten Iterationsschritt können mit der ermittelten Zentralstrahllage und dem ermittelten Kugelmittelpunkt des Messkörpers die Symmetrielinien des Bestrahlungsfeldes und des kugelförmigen Messkörpers bestimmt werden. Bei mittels eines Rundcollimators begrenzten Bestrahlungsfeldern sowie bei der Bestimmung des Kugelmittelpunktes des Messkörpers sind die Symmetrielinien mit den Schnittlinien der Messebenen identisch. Die oben beschriebene Analyse der Dosisprofile beziehungsweise die Zuordnung von Wendepunkten zu Körpergrenzen und Feldgrenzen liefert die Abstände des Zentralstrahldurchstoßpunktes und des Kugelmittelpunktes des Messkörpers in der Bildebene relativ zum EPID-Zentrum.

Es hat sich gezeigt, dass mit dem erfindungsgemäßen Verfahren bei der Definition der Feldgröße, der Zentralstrahllage, des Kugeldurchmessers sowie des Mittelpunkts des kugelförmigen Messkörpers ein Ortsauflösungsvermögen von 0.01 mm für die räumliche Abweichung des Zentralstrahls vom kugelförmigen Messkörper erreicht werden kann, was messtechnisch nachweisbar ist. Beispielsweise konnte eine verfahrensbedingte Auflösung von 0.01 mm bei einer Verwendung eines EPID mit einer Pixelgröße von 0.392 mm erreicht werden. Dies kann dadurch erreicht werden, dass ein Abschnitt der Wendetangente des Dosisprofils im Bereich des 50%-Dosispunktes zur exakten Bestimmung der Feldgrenzen beziehungsweise der Körpergrenzen des kugelförmigen Messkörpers betrachtet wird. Die Wendetangente ist in diesem Bereich des Dosisprofils die beste Approximation der Dosisverläufe über den Ortskoordinaten. Die Approximation mit dem kleinsten Fehler kann mit der kürzesten Tangente, die im digitalen Dosisbild mit diskreter Pixelgröße noch darstellbar ist, erreicht werden. In Richtung der Ortskoordinate misst sie genau ein Pixel - oder als Sonderfall zwei Pixel, wenn der 50%-Dosispunkt genau mit einem Pixelzentrum zusammenfällt. Dabei werden die beiden Pixel im Bereich des 50%-Dosispunktes ermittelt, von denen eines einen geringeren und eines einen größeren Grauwert als der 50%-Dosispunkt besitzt. Dabei kann bei Kenntnis der Pixelgröße ein konkreter Abstand ermittelt werden. Die exakten Ortskoordinaten für die Feldgrenzen des Bestrahlungsfeldes und die Körpergrenzen des kugelförmigen Messkörpers können durch lineare Interpolation zwischen den beiden ausgezeichneten Pixeln erreicht werden.

Gemäß der vorstehend erläuterten vorteilhaften Ausführungsvariante des erfindungsgemäßen Verfahrens kann es vorgesehen sein, dass der/die Wendepunkt/e im Bereich des 50%-Dosispunktes vorzugsweise zwischen zwei Pixeln festgelegt wird/werden, von denen ein erstes Pixel eine geringere Dosis als 50% und ein an das erste Pixel angrenzendes zweites Pixel eine größere Dosis als 50% repräsentiert.

Abschließend wird der Differenzvektor als Verschiebungsvektor zwischen dem Zentralstrahldurchstoßpunkt und dem Mittelpunkt des kugelförmigen Messkörpers, gemessen in der EPID-Ebene, mittels einer zentrischen Streckung in die Isozentrumsebene abgebildet, wobei das Streckzentrum der Strahlenfokus ist und der Streckfaktor gemäß

$$k = \frac{SAD}{SID} < 1 \;\; \text{mit} \;\; SAD = 1 \; m \qquad\qquad \text{Gleichung (1)}$$

von der Konstanten "Fokus-Achsabstand" SAD und der Variablen "Fokus-EPID-Abstand" SID abhängt. Außerdem werden die vom Tragarmwinkel abhängigen Ortsvektoren mittels einer Koordinatentransformation aus der Bildebene ins raumfeste Koordinatensystem des Strahlentherapiegeräts abgebildet. Die Orientierung dieses Inertialsystems ist in der Norm IEC 61217 festgeschrieben.

Nach einer weiteren Konzeption der Erfindung werden die Ortsvektoren der räumlichen Isozentren zur Kalibrierung eines Patientenpositionierungssystems eingesetzt. Alternativ oder zusätzlich kann es vorgesehen sein, dass die Ortsvektoren der räumlichen Isozentren zur Korrektur der Projektionseinrichtung eingesetzt wird, um eine Anpassung der Laserprojektion des radiologischen Isozentrums vornehmen zu können. Unter dem Ortsvektor des Isozentroids ist die räumliche Mittelpunktlage des Isozentroids zu verstehen.

Der Vorteil des erfindungsgemäßen Verfahrens liegt insbesondere in der Extraktion eines Dosisprofiles mit der Längeneinheit Millimeter aus einem Dosisbild mit der Längeneinheit Pixel, so dass dieses mit geringerem rechentechnischen Aufwand in kürzerer Zeit und insbesondere mit rechnerisch höherem Auflösungsvermögen verarbeitet werden kann. Vorteilhafterweise wird dabei ausgenutzt, dass eine Mehrzahl der bekannten Strahlentherapiegeräte ohnehin über eine Möglichkeit zur digitalen Aufnahme (EPID) eines Bestrahlungsfeldes verfügen, sodass eine Isozentrumsverifikation und eine entsprechende Korrektur mit Bestandsgeräten mit geringem Aufwand durchgeführt werden kann. So ist es wegen des vergleichsweise geringen Zeitaufwandes zur Durchführung und der automatischen Analyse des erfindungsgemäßen Verfahrens möglich, dass eine Isozentrumsverifikation (Überprüfung des Isozentrums) unmittelbar vor einer radiochirurgischen Anwendung durchgeführt werden kann, um eine bestmögliche Patientensicherheit zu gewährleisten.

Weiter hat es sich gezeigt, dass nach dem erfindungsgemäßen Verfahren ein rechnerisches Auflösungsvermögen von 0.01 mm erreicht werden kann, ohne dass gerätetechnische Veränderungen, wie etwa hochauflösende Aufnahmesysteme, erforderlich sind. Zusammenfassend kann mit einer nach dem erfindungsgemäßen Verfahren durchgeführten Isozentrumsverifikation eine erhöhte Patientensicherheit mit bereits bestehenden Strahlentherapiegeräten mit EPID erreicht werden. Da für die Durchführung der Isozentrumsverifikation und Isozentrumskorrektur nach dem erfindungsgemäßen Verfahren vergleichsweise weniger Zeit benötigt wird, können zudem Kosten eingespart werden.

Zur weiteren Optimierung kann es gemäß einer Weiterbildung des erfindungsgemäßen Verfahrens vorgesehen sein, dass eine Korrektur von Maschinentoleranzen vorgenommen wird. So kann beispielsweise eine Korrektur der Ortsvektoren $_{EPID}\mathbf{r'}_i$ im EPID-Koordinatensystem vorgesehen sein. Bei einer Fehlpositionierung des EPID kann zur Korrektur der Ortsvektoren $_{EPID}\mathbf{r'}_i$ ein weiterer Transformationsschritt vom verschobenen EPID-Koordinatensystem in das ideale EPID-Koordinatensystem erforderlich sein:

$$_{EPID}\mathbf{r}_i = \mathbf{A}_{korr} \cdot {}_{EPID}\mathbf{r}'_i,$$ 

Gleichung (2)

wobei sich die Korrekturmatrix

$$\mathbf{A}_{korr} = \mathbf{A}_\gamma \cdot \mathbf{A}_\beta \cdot \mathbf{A}_\alpha \in \mathbb{R}^{3,3}$$ 

Gleichung (3)

als Produkt aus drei Elementardrehungen zusammensetzt. Gemäß der Rechenvorschrift "Vektoren mit Matrizen von links multiplizieren" ist die Drehreihenfolge wie bei Kardanwinkeln festgelegt: Winkel $\alpha$ um die X-Achse, Winkel $\beta$ um die Y-Achse und Winkel $\gamma$ um die Z-Achse. Da bei einem Strahlentherapiegerät nach seiner Abnahmeprüfung alle Winkelfehler $\leq 1°$ sind, hat die Drehreihenfolge keinen Einfluss auf die Korrektur, und es gilt für einen allgemeinen Winkel $\alpha$: $\sin \alpha \approx \tan \alpha \approx \alpha \wedge \cos \alpha \approx 1$ mit $\alpha$ im Bogenmaß. Für eine Drehung um die X-Achse des ideal positionierten EPID ist die rechte Abbildungsmatrix in Gleichung (3)

$$\mathbf{A}_\alpha = \begin{pmatrix} 1 & 0 & 0 \\ 0 & \cos\alpha & -\sin\alpha \\ 0 & \sin\alpha & \cos\alpha \end{pmatrix}$$ 

Gleichung (4)

eine Funktion der Winkelabweichung $\alpha$. Entsprechend lauten in Gleichung (3) die Drehmatrizen um die Y-Achse und Z-Achse

$$\mathbf{A}_\beta = \begin{pmatrix} \cos\beta & 0 & \sin\beta \\ 0 & 1 & 0 \\ -\sin\beta & 0 & \cos\beta \end{pmatrix}, \qquad \mathbf{A}_\gamma = \begin{pmatrix} \cos\gamma & -\sin\gamma & 0 \\ \sin\gamma & \cos\gamma & 0 \\ 0 & 0 & 1 \end{pmatrix},$$ 

Gleichung (5)

die von den entsprechenden Winkelabweichungen $\beta$ und $\gamma$ abhängen. Die Definition der Kardanwinkel sowie die Abbildungsmatrizen in Gleichung (4) und (5) findet man beispielsweise in "Multibody dynamics with unilateral contacts" Pfeiffer, F., Glocker, Ch. (Wiley series in nonlinear science, series editors: Nayfeh, A. H. and Holden, A. V., John Wiley & Sons, Inc., New York, 1996). Translatorische Korrekturen müssen dabei nicht angewendet werden, da der gesuchte Differenzvektor

$$\mathbf{r}_{ISO} = \mathbf{r}_{CAX} - \mathbf{r}_{WLP} \in \mathbb{R}^3$$ 

Gleichung (6)

zwischen dem Durchstoßpunkt $\mathbf{r}_{CAX}$ des Zentralstrahls und dem Kugelmittelpunkt $\mathbf{r}_{WLP}$ des Messkörpers nicht von Verschiebungen des EPID in seiner X-Y-Ebene abhängen. Die Toleranz der Vertikalkoordinate wird bei der zentrischen Streckung von der Bildebene in die Isozentrumsebene durch den korrigierten Fokus-EPID-Abstand in Gleichung (1) berücksichtigt. Die Toleranzen der drei Winkelfreiheitsgrade können ebenfalls berücksichtigt werden. Abweichungen, die für den Tragarmwinkel, den Collimatorwinkel, und den Tischwinkel gemessen werden, können bei Anwendung einer entsprechenden Software durch Eingabe berücksichtigt werden, wobei sie als Teil eines Algorithmus zur Fehlerkorrektur beitragen.

Bei der Radiochirurgie und stereotaktischen Präzisionsbestrahlung werden wegen unregelmäßiger Strukturen des zu bestrahlenden Gewebes üblicherweise keine rechteckigen beziehungsweise quadratischen Bestrahlungsfelder eingesetzt. Für solche Fälle werden die Bestrahlungsfelder mittels der Lamellen eines Multi Leaf Collimators (MLC) begrenzt. Nach einer Ausführungsvariante des erfindungsgemäßen Verfahrens, bei der ein MLC zum Einsatz kommt, werden daher die Zentralstrahllagen zwischen mehreren Lamellenpaaren eines MLC in X-Richtung bestimmt. So kann es vorgesehen sein, dass bei der Verwendung eines MLC die Schritte d) bis h) des erfindungsgemäßen Verfahrens für jedes das Bestrahlungsfeld begrenzende Lamellenpaar des MLC durchgeführt werden. Dabei kann es weiter vorgesehen sein, dass die äußeren Lamellenpaare nicht zur Analyse herangezogen werden, da dort die Dosisgradienten durch Streuphotonen vom Blendensystem zur Feldbegrenzung in Y-Richtung gestört sind.

Zur Bestimmung der Zentralstrahllage in Y-Richtung werden bei Bestrahlungsfeldern, die durch Blockblenden oder den MLC geformt werden, zwei Dosisprofile ausgewertet, die spiegelbildlich zur Symmetrielinie der X-Koordinate ausgeschnitten werden. Die spiegelsymmetrischen Messebenen sowie die arithmetische Mittelung der einzelnen Zentralstrahllagen in beiden Richtungen X und Y haben den Vorteil, dass sich eventuelle Winkelfehler des Collimators im Ergebnis durch die Mittelung ausgleichen. Ein weiterer Vorteil besteht darin, dass weniger Feldstörungen durch den Messkörper hervorgerufen werden, da die interessierenden 50%-Dosispunkte der Dosisprofile einen größeren Abstand vom Messkörper beziehungsweise vom Winston-Lutz-Pointer aufweisen.

Gemäß einer weiteren vorteilhaften Ausführungsvariante des erfindungsgemäßen Verfahrens wird bei einer Variation des Patiententischwinkels ein Tragarmwinkel $\neq 0°$ eingestellt, um eine Aussage über die vertikale Positionsänderung des Messkörpers zu erhalten. Vorzugsweise wird bei einer Variation des Winkels des Patientenlagerungstischs ein Tragarmwinkel von 30° eingestellt, wobei ein Winkel des Patientenlagerungstischs im Bereich zwischen 0° und 90° ermöglicht ist. Gleichermaßen ermöglicht ein Tragarmwinkel von -30° einen Winkel des Patientenlagerungstischs in einem Bereich zwischen 0° und -90°. Für den Fall, dass ein raumfestes, von dem Strahlentherapiegerät unabhängiges Patientenpositionierungssystem eingesetzt wird, kann das Isozentroid des Tisches alternativ ohne Bewegung des Tragarms ermittelt werden. Bei der vorstehenden Variante wird die Tischwinkelvariation mit den diskreten Winkeln (-90 : 30 : 90)° durchgeführt. Die Stellung des Tragarms beträgt dabei 0°, sodass sich der Strahlerkopf nicht in den beiden Nutzstrahlenfeldern des externen Röntgensystems befindet. Für den Fall, dass das Patientenpositionierungssystem ExacTrac 6.0.6 (BRAINLAB AG, Feldkirchen, Deutschland) eingesetzt wird, wird bei jedem eingestellten Tischwinkel die räumliche Position des Messkörpers, bei dem es sich um einen Winston-Lutz-Pointer der Firma BRAINLAB AG (Feldkirchen, Deutschland) handeln kann, im Verifikationsschritt *Detect Winston-Lutz Pointer* mit der Funktionalität *Winston-Lutz Pointer Analysis* radiologisch ermittelt. Die negativen Werte der Verschiebungen des Messkörpers relativ zum idealen Isozentrum des Strahlentherapiegeräts werden vektoriell in der Datei WL_Test.log gespeichert. Dabei handelt es sich um Daten in Form eines Textdokuments, welches vom Softwarepaket MATLAB® gelesen werden kann. In den Daten der Datei geht aus den Spalten 15-17 der tischwinkelabhängige Verschiebungsvektor

$$[-WLshiftLat \, . \quad -WLshiftLong \, . \quad -WLshiftVert] \qquad \text{Gleichung (7)}$$

hervor, der im Initialsystem die Form

$$\mathbf{r}_{ET}(T) = \begin{pmatrix} X_{ET} \\ Y_{ET} \\ Z_{ET} \end{pmatrix} = - \begin{pmatrix} WLshiftLat \\ WLshiftLong \\ WLshiftVert \end{pmatrix} \qquad \text{Gleichung (8)}$$

aufweist. Der Offset

$$r_0 = \begin{pmatrix} \Delta X_{CAX}(G = 0° \wedge T = 0°) \\ \Delta Y_{CAX}(G = 0° \wedge T = 0°) \\ \Delta Z_{CAX}(G = 0° \wedge T = 0°) \end{pmatrix} \qquad \text{Gleichung (9)}$$

des Zentralstrahls vom idealen Isozentrum des Strahlentherapiegeräts ist von der Bestimmung des tragarmwinkelabhängigen Isozentroids bekannt und ändert sich nicht mit dem Tischwinkel. Somit ist der Verschiebungsvektor (Abstandsvektor) des Zentralstrahls vom Messkörper die Vektorsumme

$$\Delta r_{CAX}(T) = r_{ET}(T) + r_0 \ , \qquad \text{Gleichung (10)}$$

womit der Isozentroid des Patientenlagerungstischs im Inertialsystem beschrieben wird.

Gemäß einer Weiterbildung des erfindungsgemäßen Verfahrens kann aus den einzelnen Abweichungen in den drei Raumrichtungen X, Y und Z ein globaler Isozentroid bestimmt werden. Dabei wird wie folgt vorgegangen: Nachdem für jeden Winkelfreiheitsgrad die Geometrie des Isozentroids mit den Kennmerkmalen

- Abweichungen des Zentralstrahls vom Winston-Lutz-Pointer in den drei Raumrichtungen X, Y und Z als Funktion eines Winkels,
- radiale betragsmäßige Abweichung des Zentralstrahls vom Winston-Lutz-Pointer als Funktion eines Winkels,
- Ortskoordinaten des Isozentroids,
- maximale Durchmesser des Isozentroids in den drei Raumrichtungen und
- maximaler globaler Radius des Isozentroids

im raumfesten Koordinatensystem (Inertialsystem) ermittelt wurde, lässt sich mit allen Ergebnissen ein globaler Isozentroid bestimmen. Dies kann erreicht werden, indem die winkelabhängigen Messbedingungen zusammengefasst betrachtet werden:

- Das Isozentroid des Tragarms wird bei 0° Collimator- und Tischwinkel ermittelt.
- Das Isozentroid des Collimators wird bei 0° Tragarm- und Tischwinkel ermittelt.
- Das Isozentroid des Tisches wird jeweils zur Hälfte mit 30° beziehungsweise -30° Tragarmwinkel sowie mit 0° Collimatorwinkel ermittelt.

Basierend auf der Isozentroiddarstellung als Funktion des Tragarmwinkels lassen sich die zusätzlichen maximal möglichen Isozentrumsabweichungen infolge der Collimatorwinkelvariation und Tischwinkelvariation als Streuungsbänder in positiver und negativer Richtung über dem Tragarmwinkel darstellen. Die Auswertung bezüglich der Ortskoordinaten und maximalen Durchmesser beziehungsweise des größten Radius wird abschließend aktualisiert. Außerdem werden die Achsabstände, die gemäß der Norm DIN 6875 - Teil 2 mindestens halbjährlich zu messen sind, zwischen allen drei Rotationsachsen ermittelt. Die Streuungsbänder haben eine negative und positive Komponente. Ihre Bestimmung erfordert für die Isozentrumsabweichungen der Collimatorwinkelvariation zuerst eine Abbildung vom EPID-Koordinatensystem ins initiale Koordinatensystem:

$$_I r_{ISO}(G, C) = A_\beta \cdot {}_{EPID} r_{ISO}(C) \ . \qquad \text{Gleichung (11)}$$

Die Abbildungsmatrix ist aus Gleichung (5) bekannt, wobei $\beta = G$ der Tragarmwinkel ist. Der Ortsvektor

$$\mathbf{r}_{ISO} = \begin{pmatrix} X_{ISO} \\ Y_{ISO} \\ Z_{ISO} \end{pmatrix} \qquad \text{Gleichung (12)}$$

setzt sich aus den Komponenten der Isozentrumsabweichung zusammen. Die negativen Streuungsbänder ergeben sich zu

$$\Delta X_{ISO}^{-}(G) = \min_{C \in [-175°, +175°]} [X_{ISO}(G, C)] - X_{ISO}(G, C = 0°) \ , \quad \text{Gleichung (13)}$$

$$\Delta Y_{ISO}^{-}(G) = \min_{C \in [-175°, +175°]} [Y_{ISO}(G, C)] - Y_{ISO}(G, C = 0°) \ , \quad \text{Gleichung (14)}$$

$$\Delta Z_{ISO}^{-}(G) = \min_{C \in [-175°, +175°]} [Z_{ISO}(G, C)] - Z_{ISO}(G, C = 0°) \ . \quad \text{Gleichung (15)}$$

Entsprechend erhält man die positiven Streuungsbänder gemäß

$$\Delta X_{ISO}^{+}(G) = \max_{C \in [-175°, +175°]} [X_{ISO}(G, C)] - X_{ISO}(G, C = 0°) \ , \quad \text{Gleichung (16)}$$

$$\Delta Y_{ISO}^{+}(G) = \max_{C \in [-175°, +175°]} [Y_{ISO}(G, C)] - Y_{ISO}(G, C = 0°) \ , \quad \text{Gleichung (17)}$$

$$\Delta Z_{ISO}^{+}(G) = \max_{C \in [-175°, +175°]} [Z_{ISO}(G, C)] - Z_{ISO}(G, C = 0°) \ . \quad \text{Gleichung (18)}$$

Vorzugsweise kann das Intervall des Collimatorwinkels für die Extremasuche in den Gleichungen (13) bis (18) mit C $\in$ [-90°, +90°] definiert werden, da alle optimalen Collimatorwinkel bei der Patientenbehandlung in diesem Bereich liegen. Die Streuungsbänder bei der Tischwinkelvariation ergeben sich analog zu den Gleichungen (13) bis (18), indem man den Collimatorwinkel C durch den Tischwinkel T und die Intervalle zur Extremasuche mittels T $\in$ [-90°, +90°] definiert.

Im Rahmen des erfindungsgemäßen Verfahrens können Isozentroide durch Optimierung minimiert werden. Eine Minimierung durch Optimierung ist denkbar für das

- Isozentroid des Tragarms für den Fall, dass ein Rundcollimator und ein externes Patientenpositionierungssystem eingesetzt wird,

- kombinierte Isozentroid des Tragarms und des Collimators für den Fall, dass ein Patientenpositionierungssystem eingesetzt wird,

- kombinierte Isozentroid des Tragarms und des Patiententisches für den Fall, dass ein Rundcollimator ohne Patientenpositionierungssystem eingesetzt wird, und/oder

- globale Isozentroid für den Fall, dass weder ein Rundcollimator noch ein Patientenpositionierungssystem zur Verfügung steht.

Sofern in Verbindung mit dem Strahlentherapiegerät ein unabhängiges radiologisches Patientenpositionierungssystem verwendet wird, kann das Isozentroid des Patientenlagerungstisches unbeachtet bleiben, da von diesem alle tischwinkelabhängigen Zentralstrahlabweichungen erkannt und korrigiert werden. Beim Einsatz von Rundcollimatoren gilt für den Collimatorwinkel C üblicherweise C = 0° = konstant.

Bei der Feldbegrenzung mit einem MLC können die Optimierungsvariablen im Vektor

$$\mathbf{x}_O = \begin{pmatrix} x_{CLO} \\ y_{JO} \\ 0 \end{pmatrix} \in \mathbb{R}^3 \qquad \text{Gleichung (19)}$$

zusammengefasst werden, wobei $x_{CLO}$ der Centreline Offset der Lamellen und $y_{JO}$ der Jaw Offset des Y-Blendenpaares ist. Die erste Komponente dieses Vektors kann auch der Jaw Offset des X-Blendenpaares sein, sofern damit das Bestrahlungsfeld begrenzt werden soll. Die zweite Komponente kann auch mit 0 identisch sein, wenn die Feldbegrenzung in Y-Richtung allein durch die Lamellen des MLC erreicht wird. Bei der Verwendung von Rundcollimatoren beinhaltet Gleichung (19) deren Offsets. Die oben aufgeführten Isozentroide, das heißt das Isozentroid des Tragarms, das kombinierte Isozentroid des Tragarms und des Collimators, das kombinierte Isozentroid des Tragarms und des Patiententisches und das globale Isozentroid, sind Funktionen des Tragarmwinkels G. Das solitäre Tragarmisozentroid ist aus den Komponenten

$$\mathbf{r}_{ISO}(G) = \begin{pmatrix} X_{ISO}(G) \\ Y_{ISO}(G) \\ Z_{ISO}(G) \end{pmatrix} \qquad \text{Gleichung (20)}$$

zusammengesetzt.

Die kombinierten Isozentroide können in jeder Raumrichtung durch zwei Funktionen beschrieben werden, die sie in die negative Richtung beziehungsweise in die positive Richtung begrenzen:

$$\mathbf{r}_{ISO}^-(G) = \begin{pmatrix} X_{ISO}^-(G) \\ Y_{ISO}^-(G) \\ Z_{ISO}^-(G) \end{pmatrix}, \ \mathbf{r}_{ISO}^+(G) = \begin{pmatrix} X_{ISO}^+(G) \\ Y_{ISO}^+(G) \\ Z_{ISO}^+(G) \end{pmatrix} \forall C, T \in [-90°, +90°] \quad . \ \text{Gleichung (21)}$$

Der Collimatorwinkel C und der Tischwinkel T des Patientenlagerungstischs können dabei im angegebenen Intervall variiert werden. Der Wertebereich des Collimatorwinkels C kann mit ± 90° beschränkt werden, da üblicherweise alle optimalen Collimatorwinkel in diesem Bereich liegen.

Nach einer weiteren vorteilhaften Ausführungsvariante des erfindungsgemäßen Verfahrens kann es vorgesehen sein, dass sämtliche die räumlichen Isozentren beeinflussende gerätespezifische Parameter durch Anwendung einer/mehrerer vorgegebenen/er Zielfunktion/en optimiert werden. So kann eine Korrektur von Maschinenparametern vorgesehen sein. Vorteilhafterweise kann die Optimierung mittels einer Software durchgeführt werden. Mögliche zur Optimierung einsetzbare Zielfunktionen, die von den Variablen in Gleichung (19) abhängen, betreffen die Summen, arithmetischen Mittelwerte, Extrema und Integrale über dem Tragarmwinkel der Quadrate der

- Abstände $X_{ISO}^-(G_i)$, $Y_{ISO}^-(G_i)$, $Z_{ISO}^-(G_i)$, $X_{ISO}^+(G_i)$, $Y_{ISO}^+(G_i)$, $Z_{ISO}^+(G_i)$,
- räumlichen Radien

$$R_{ISO}(G_i) = \sqrt{X_{extr}^2(G_i) + Y_{extr}^2(G_i) + Z_{extr}^2(G_i)}$$

,
- Durchmesser $D_X(G_i)$, $D_Y(G_i)$ und $D_Z(G_i)$

des untersuchten Isozentroids bei diskreten Tragarmwinkeln $G_i \in [-180°, +180°]$.

Für die Komponenten des räumlichen Radius gilt:

$$K_{extr}(G_i) = \max\left[\left|\min\left[K_{ISO}^-(G_i)\right]\right|, \left|\max\left[K_{ISO}^+(G_i)\right]\right|\right] \text{ mit } K \in \{X, Y, Z\} \quad \text{Gleichung (22)}$$

beim diskreten Tragarmwinkel $G_i$.

Die Definition der Durchmesser lautet

$$D_K(G_i) = K_{ISO}^+(G_i) - K_{ISO}^-(G_i) \text{ mit } K \in \{X, Y, Z\} \quad . \text{ Gleichung (23)}$$

Im Spezialfall des solitären Isozentroids des Tragarms sind die geometrischen Größen, die optimiert werden können, wie folgt definiert:

- Die Abstände $X_{ISO}(G_i)$, $Y_{ISO}(G_i)$ und $Z_{ISO}(G_i)$.
- Die räumlichen Radien

$$R_{ISO}(G_i) = \sqrt{X_{ISO}^2(G_i) + Y_{ISO}^2(G_i) + Z_{ISO}^2(G_i)} \ .$$

- Die maximalen Durchmesser

$$D_K(G_i) = \max_{G_i}\left[K_{ISO}(G_i)\right] - \min_{G_i}\left[K_{ISO}(G_i)\right]$$

mit $K \in \{X, Y, Z\}$. Mit den drei geometrischen Größen und jeweils vier Funktionen können somit zwölf verschiedene Zielfunktionen zur Optimierung gewählt werden, wobei einige Zielfunktionen bezüglich der Optimierung redundant sind:

$$\sum_{K \in \{X,Y,Z\}}\left[\int_{G_i}\left[K_{ISO}^+(G_i) - K_{ISO}^-(G_i)\right]^2 dG\right] \equiv \sum_{K \in \{X,Y,Z\}}\left[\int_{G_i} D_K^2(G_i)\, dG\right],$$

$$\sum_{G_i} R_{ISO}^2(G_i) \equiv \operatorname*{mean}_{G_i}\left[R_{ISO}^2(G_i)\right] \ ,$$

$$\sum_{G_i}\left[D_X^2(G_i) + D_Y^2(G_i) + D_Z^2(G_i)\right] \equiv \sum_{K \in \{X,Y,Z\}}\left[\operatorname*{mean}_{G_i}\left[D_K^2(G_i)\right]\right] \ .$$

Bei der Suche nach der für eine konkrete Bestrahlungsaufgabe besten Optimierung ist es daher nicht erforderlich, sämtliche Zielfunktionen zu betrachten, da die Funktionen jedes der hier genannten Paare identische Ergebnisse liefern.

Während der Optimierung wird der Ortsvektor der Zentralstrahlabweichungen in Gleichung (6) gemäß

$$\mathrm{r}'_{CAX} = \mathrm{r}_{CAX} + \mathbf{x}_O \in \mathbb{R}^3 \qquad\qquad \text{Gleichung (24)}$$

im Detektorkoordinatensystem (EPID-Koordinatensystem) variiert. Mit Hilfe der Gleichungen (5) und (6) können die Ortsabweichungen der untersuchten Isozentroide im raumfesten Koordinatensystem des Strahlentherapiegeräts bestimmt werden. Dabei sind in Gleichung (5) die Winkel $\beta$ = G und $\gamma$ = C durch den Tragarmwinkel beziehungsweise den Collimatorwinkel zu ersetzen.

Der Vektor $\mathbf{x}_0$ ist dann optimal, wenn für eine der oben skizzierten Zielfunktionen, die die Variablen der Gleichung (19) in quadratischer Form enthält, gilt:

$$f(\mathbf{x}_O) = \text{Minimum}! \qquad\qquad \text{Gleichung (25)}$$

Zur Justierung der Collimatorparameter an einem Strahlentherapiegerät können dann die optimierten Parameter in Gleichung (19) aus den verschiedenen Lösungen ausgewählt werden. Beispielsweise können diejenigen ausgewählt werden, die den mittleren räumlichen Radius

$$\int_{G_i} R_{ISO}(G_i)\, dG \qquad\qquad \text{Gleichung (26)}$$

des Zentralstrahls minimieren. Für die Systematik proportionaler, identischer Sicherheitsräume in allen Raumrichtungen, mit der ein Tumorvolumen auf das Bestrahlungsvolumen erweitert wird, sodass ein Tumor auch mit Lagerungsungenauigkeiten, Tumorbeweglichkeiten und Maschinentoleranzen vollständig bestrahlt werden kann, würde lediglich der maximale Durchmesser des relevanten Isozentroids minimiert werden:

$$\max_{G_i} \left[ D_X(G_i), D_Y(G_i), D_Z(G_i) \right] . \qquad\qquad \text{Gleichung (27)}$$

Zur Lösung der nichtlinearen Optimierungsaufgabe ohne Nebenbedingungen in Gleichung (25) kann die Subroutine fminsearch in MATLAB® eingesetzt werden. Das dabei verwendete Rechenschema ist ein Simplex-Algorithmus des Typs Nelder-Mead zur direkten Minimumsuche.

Weiterhin kann es nach einer vorteilhaften Ausführungsvariante des erfindungsgemäßen Verfahren vorgesehen sein, dass geometrische Toleranzen des Strahlentherapiegeräts quantifiziert und rechnerisch berücksichtigt werden.

Nach einer weiteren vorteilhaften Ausführungsvariante des erfindungsgemäßen Verfahrens kann es vorgesehen sein, dass das Bestrahlungsfeld unter Berücksichtigung von geeigneten Werten einer Feldgröße, einer Relaxationszeit des Tragarms, einer energieabhängigen Dosis je Bestrahlungsfeld, und/oder eines Fokus-EPID-Abstandes appliziert wird.

Weiterhin kann eine Anwendung des erfindungsgemäßen Verfahrens an einem Therapiesimulator vorgesehen sein, wobei die räumlichen Isozentren bestimmt, korrigiert und minimiert werden.

Anhand der nachfolgenden Figuren soll die Erfindung beispielhaft näher erläutert werden.

Es zeigen:

Figur 1     ein zweidimensionales Dosisbild eines kugelförmigen Messkörpers in einem MLC-begrenzten Bestrahlungsfeld,

Figur 2a    ein Dosisprofil eines zweidimensionalen Dosisbildes zur Erläuterung eines Ausführungsbeispiels des erfindungsgemäßen Verfahrens,

Figur 2b    das Dosisprofil aus Figur 2a zur Erläuterung der Definition der 50%-Dosispunkte,

Figur 3     eine schematische Darstellung zum messtechnischen Nachweis des erreichten Ortsauflösungsvermögens,

Figur 4     Dosisprofile des Winston-Lutz-Pointers als Messkörper,

Figur 5     weitere Dosisprofile eines MLC-begrenzten Bestrahlungsfeldes in X-Richtung,

Figur 6     weitere Dosisprofile eines MLC-begrenzten Bestrahlungsfeldes in Y-Richtung,

Figur 7     eine schematische Darstellung der Geometrie eines tragarmwinkelabhängigen Isozentroids eines Strahlentherapiegeräts,

Figur 8     eine grafische Darstellung eines globalen Isozentroids.

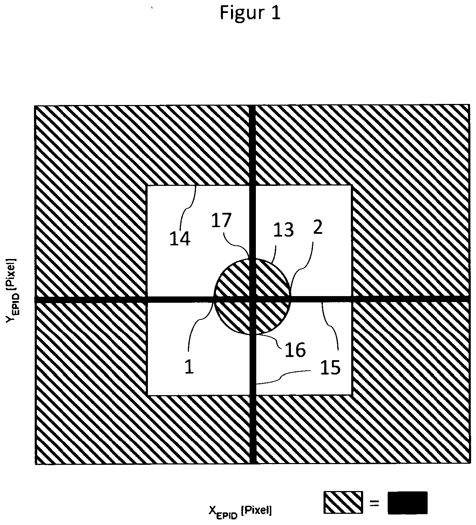

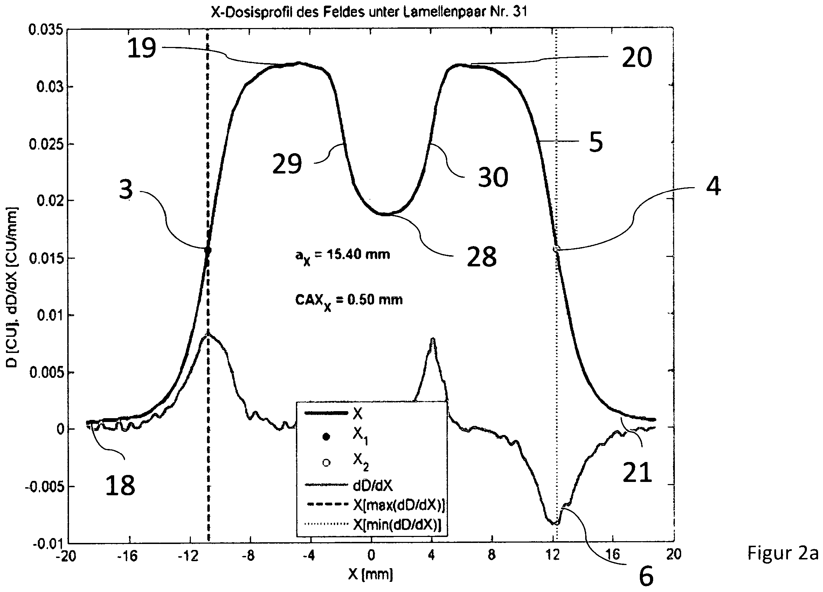

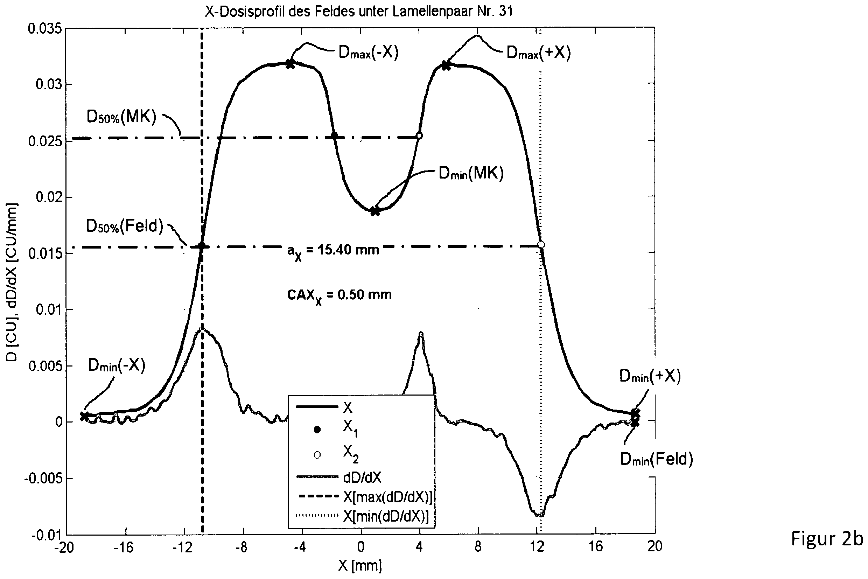

Zur Erläuterung des erfindungsgemäßen Verfahrens sind die Figuren 1, 2a und 2b gemeinsam zu betrachten.

Bei dem erfindungsgemäßen Verfahren zur EPID-basierten Überprüfung und Korrektur des Isozentrums eines Strahlentherapiegeräts, welches mindestens einen um mindestens eine Tischachse drehbaren Patientenlagerungstisch, einen um eine Tragarmachse drehbaren Tragarm, einen an dem Tragarm angeordneten Strahlerkopf zur Applikation des Therapiestrahls, einen Collimator zur Begrenzung eines Strahlenfeldes, eine Einrichtung zum Projizieren des radiologischen Isozentrums und ein digitales Aufnahmesystem (EPID) zur Erzeugung von Dosisbildern mittels des Therapiestrahls umfasst, wird nach den folgenden Schritten vorgegangen:

a) Ein Messkörper 13 wird an der Projektionsposition des radiologischen Isozentrums eines Strahlentherapiegeräts positioniert,

b) anschließend wird ein Bestrahlungsfeld für mindestens eine vorgegebene Winkeleinstellung des Tragarms, des Patientenlagerungstischs und des Collimators appliziert und dabei

c) mit dem EPID ein Dosisbild des Messkörpers 13 aufgenommen, wie es in Figur 1 dargestellt ist. Bei dem Dosisbild in Figur 1 handelt es sich um ein zweidimensionales Graustufen-Dosisbild eines MLC-begrenzten Bestrahlungsfeldes der Größe $15 \times 15\ mm^2$ in MATLAB® (Version R2007a). Das verwendete EPID weist Pixel mit einer Kantenlänge von 0.392 mm auf. Das Bezugszeichen 13 kennzeichnet einen kugelförmigen Messkörper (Winston-Lutz-Pointer) mit bekanntem Durchmesser. Das Bezugszeichen 14 kennzeichnet eine durch den Collimator hervorgerufene Grenze des Bestrahlungsfeldes. Im Dosisbild weisen dunkle Bereiche eine gegenüber hellen Bereichen geringere applizierte Energiedosis auf. Zur Bestimmung des Kugelmittelpunkts des Messkörpers 13 mittels der gekreuzten, orthogonalen Messebenen 15 werden zunächst beide Kugelgrenzen 1 und 2 in X-Richtung und beide Kugelgrenzen 16 und 17 in Y-Richtung bestimmt. Die Kugelgrenzen 1, 2, 16 und 17 können aus den Dosisprofilen ermittelt werden, welche anhand des aufgenommenen Dosisbilds in Schritt d) des Verfahrens erstellt werden. Figur 2a und Figur 2b zeigen ein Dosisprofil 5 in X-Richtung des in Figur 1 gezeigten Dosisbildes. Die mit dem Bezugszeichen 6 in Figur 2a gekennzeichnete Kurve repräsentiert den Steigungsverlauf des Dosisprofils über der X-Achse.

Weiter in Schritt e) des Verfahrens wird zunächst eine Mittelpunktlage des Messkörpers 13 bestimmt, indem im Verlauf des Dosisprofils 5 an zumindest einer zu erwartenden Grenze des Messkörpers 13 zwischen einem Dosismaximum 19 und einem Dosisminimum 28 ein Wendepunkt 29 und zwischen einem Dosisminimum 28 und einem Dosismaximum 20 ein Wendepunkt 30 ermittelt wird. Die Bestimmung der Wendepunkte 29 und 30 kann im Bereich des 50%-Dosispunktes erfolgen, wobei es sich bei dem 50%-Dosispunkt um die Position im Verlauf des Dosisprofils 5 handelt, bei der die Dosis zwischen dem Dosisminimum 28 und den Dosismaxima 19 und 20 50% aufweist. Vorzugsweise wird der 50%-Dosispunkt zwischen zwei Pixeln ermittelt, von denen ein erstes Pixel eine geringere Dosis als 50% und ein an das erste Pixel angrenzendes zweites Pixel eine größere Dosis als 50% repräsentiert.

Analog kann zur Bestimmung der Feldgrenzen des Bestrahlungsfeldes vorgegangen werden, wobei im Verlauf des Dosisprofils 5 an zumindest einer zu erwartenden Grenze des Bestrahlungsfeldes zwischen einem Dosisminimum 18 und einem Dosismaximum 19 ein Wendepunkt 3 und zwischen einem Dosismaximum 20 und einem Dosisminimum 21 ein Wendepunkt 4 ermittelt wird. Die Bestimmung der Wendepunkte 3 und 4 kann im Bereich des 50%-Dosispunktes erfolgen, wobei es sich bei dem 50%-Dosispunkt um die Position im Verlauf des Dosisprofils 5 handelt, bei der die Dosis zwischen den Dosisminima 18 sowie 21 und den Dosismaxima 19 sowie 20 50% aufweist. Vorzugsweise wird der 50%-Dosispunkt zwischen zwei Pixeln ermittelt, von denen ein erstes Pixel eine geringere Dosis als 50% und ein an das erste Pixel angrenzendes zweites Pixel eine größere Dosis als 50% repräsentiert.

Im anschließenden Schritt f) des Verfahrens werden die ermittelten Wendepunkte einer Feldgrenze beziehungsweise einer Messkörpergrenze zugeordnet. Im gezeigten Beispiel kann der Wendepunkt 29 der in negativer X-Richtung gelegenen Körpergrenze 1 des Messkörpers 13 und der Wendepunkt 30 der in positiver X-Richtung gelegenen Körpergrenze 2 des Messkörpers 13 zugeordnet werden. Gleichermaßen kann eine Bestimmung der Körpergrenzen des Messkörpers 13 in Y-Richtung anhand eines Dosisprofils in Y-Richtung erfolgen. In Figur 1 sind die Körpergrenzen in Y-Richtung mit den Bezugszeichen 16 und 17 gekennzeichnet. Der Wendepunkt 3 kann der in negativer X-Richtung gelegenen Feldgrenze und der Wendepunkt 4 der in positiver X-Richtung gelegenen Feldgrenze zugeordnet werden. Gleichermaßen kann eine Bestimmung der Feldgrenzen in Y-Richtung anhand eines Dosisprofils in Y-Richtung erfolgen.

Nach Festlegung der beiden Körpergrenzen des Messkörpers 13 kann durch die arithmetische Mittelwertbildung der beiden Ortskoordinaten der Grenzpunkte die Mittelpunktposition des Messkörpers 13 bestimmt werden. Vorzugsweise werden die Abstände der Körpergrenzen in X-Richtung und in Y-Richtung zur Bestimmung der Mittelpunktposition eingesetzt. Gleichermaßen kann nach Festlegung der beiden Feldgrenzen 3 und 4 durch die arithmetische Mittelwertbildung der Ortskoordinaten der Grenzpunkte die Zentralstrahllage bestimmt werden. Vorzugsweise werden die Abstände der Feldgrenzen in X-Richtung und in Y-Richtung zur Bestimmung des Zentralstrahlposition eingesetzt.

Im weiteren Schritt g) des Verfahrens werden anhand der zugeordneten Körpergrenzen des Messkörpers 13 sowie anhand der zugeordneten Feldgrenzen des Bestrahlungsfeldes die Lagen des Mittelpunkts des Messkörpers 13 sowie des Bestrahlungsfeldes relativ zum EPID-Zentrum ermittelt. Die Schritte d) bis g) werden für die Richtungen X und Y durchgeführt. Im Schritt h) wird der Differenzvektor in der EPID-Ebene, der vom Mittelpunkt des Messkörpers 13 zum Zentralstrahldurchstoßpunkt durch die EPID-Ebene zeigt, in die Isozentrumsebene gemäß Gleichung (1) projiziert. Die Schritte b) bis h) werden für alle vorgeschriebenen Tragarmwinkel, Collimatorwinkel und Tischwinkel (des Patientenlagerungstischs) ausgeführt.

Abschließend werden die Vektorkomponenten des Differenzvektors zur Korrektur des momentanen radiologischen Isozentrums eingesetzt.

Das erfindungsgemäße Verfahren erreicht mit einem klinisch standardmäßig eingesetzten EPID ein Ortsauflösungsvermögen von 0.01 mm, was einem 39.2-fach verbesserten Auflösungsvermögen gegenüber dem standardmäßig eingesetzten EPID entspricht.

Die Messbedingungen für das in Figur 2 dargestellte Dosisprofil wurden wie folgt festgelegt: Tragarmwinkel = 0°, Collimatorwinkel = 0°, Tischwinkel = 0°, nominelle Feldgröße = $15 \times 15$ mm$^2$, Fokus-EPID-Abstand = 1.5 m (Vergrößerungsfaktor = 1.5), Photonenenergie = 6 MeV und bestrahlte Dosis = 12 MU. Die Dosis D wird vom EPID in der Einheit [CU] (kalibrierte Dosiseinheit) gemessen, wobei unter Kalibrier-Messbedingungen 1 CU = 1 Gy gilt. Die Größen $a_x$ und $CAX_X$ sind die Feldbreite beziehungsweise Zentralstrahllage in X-Richtung. Beim gleichen Bestrahlungsfeld ohne Winston-Lutz-Pointer wurden dieselben Werte für diese Größen ermittelt. Die Grafik wurde mittels MATLAB® (Version R2007a) erzeugt.

Figur 2b zeigt die Definition aller vier 50%-Dosispunkte des Dosisprofils in X-Richtung der Figur 2a, die zur Bestimmung der Messkörpergrenzen, Bestrahlungsfeldgrenzen sowie der Positionen des Messkörpermittelpunkts und des Zentralstrahls benötigt werden. Alle 50%-Dosisniveaus werden als arithmetisches Mittel eines lokalen Dosisminimums und eines lokalen Dosismaximums definiert. Als 100%-Dosis wird das kleinste aller lokalen Dosismaxima festgelegt. Als Dosisminima werden die kleinstmöglichen Werte für den Messkörper und für das Bestrahlungsfeld eingesetzt. Wenn sowohl das jeweilige Dosismaximum als auch das jeweilige Dosisminimum zur Berechnung des 50%-Dosispunktes minimal ist, wird sein Dosiswert ebenfalls minimal. Jeder 50%-Dosispunkt liegt dabei im Bereich des dortigen Wendepunktes. Beide Aspekte erhöhen das Auflösungsvermögen des erfindungsgemäßen Verfahrens. Dass ein niedrig gelegener 50%-Dosispunkt von Vorteil ist, lässt sich physikalisch wie folgt erklären: Je tiefer der 50%-Dosispunkt des Feldes liegt, umso weniger Störeinfluss haben die Streuphotonen, die im Messkörper erzeugt werden, da der Abstand des Messkörpers zum 50%-Dosispunkt zunimmt. Und umgekehrt, je tiefer der 50%-Dosispunkt des Messkörpers liegt, umso weniger Störeinfluss haben die Streuphotonen, die durch die Feldbegrenzung (Blockblenden, Lamellen eines MLC oder einen Rundcollimator) erzeugt werden, da der Abstand der Feldbegrenzung vom 50%-Dosispunkt zunimmt. Die Figur 2b zeigt ein Dosisprofil für die Bestimmung der geometrischen Eigenschaften des Bestrahlungsfeldes und des Messkörpers in X-Richtung. Im erfindungsgemäßen Verfahren werden jedoch dafür mindestens zwei unterschiedliche Dosisprofile aus einem Dosisbild ausgeschnitten, die unterschiedliche Y-Koordinaten besitzen. In Y-Richtung wird entsprechend vorgegangen. Dies trägt ebenfalls zum guten Auflösungsvermögen beziehungsweise zur Fehlerminimierung des erfindungsgemäßen Verfahrens bei. Die Bestimmungsgleichungen lauten:

$$D_{100\%} = \min[D_{max}(-X), D_{max}(+X), D_{max}(-Y), D_{max}(+Y)],$$

$$D_{min}(Feld) = 0 \ [CU],$$

$$D_{50\%}(MK) = [D_{100\%} + D_{min}(MK)] / 2,$$

$$D_{50\%}(Feld) = [D_{100\%} + D_{min}(Feld)] / 2 = D_{100\%} / 2,$$

$$\Delta X(MK) = [X_1(MK) + X_2(MK)] / 2,$$

$$\Delta Y(MK) = [Y_1(MK) + Y_2(MK)] / 2,$$

$$\Delta CAX_x = [X_1(Feld) + X_2(Feld)] / 2,$$

$$\Delta CAX_Y = [Y_1(Feld) + Y_2(Feld)] / 2,$$

$$\Delta X_{ISO} = \Delta CAX_x - \Delta X(MK),$$

$$\Delta Y_{ISO} = \Delta CAX_Y - \Delta Y(MK).$$

Legende zu den Gleichungen:

MK = Messkörper oder Winston-Lutz-Pointer oder Wolframkugel (der in dem zur Erläuterung eines Ausführungsbeispiels des erfindungsgemäßen Verfahrens verwendete Winston-Lutz-Pointer ist ein kommerzieller der Firma BRAINLAB AG, Feldkirchen, Deutschland)

Feld = Bestrahlungsfeld mit bestimmter Feldbreite (X-Richtung) und Feldlänge (Y-Richtung)

Koordinatensystem = EPID-Koordinatensystem

$D_{100\%}$ = 100%-Ersatzdosis (vergleiche 100%-Dosis der Norm IEC 60976)

$D_{50\%}(Feld)$ = 50%-Dosis zur Bestimmung der Feldbreite und Feldlänge mittels der dort lokalisierten Wendetangente

$D_{50\%}(MK)$ = 50%-Ersatzdosis zur Bestimmung der Messkörpergrenzen mittels der dort lokalisierten Wendetangente

$D_{max}(-X)$ = lokales Dosismaximum des X-Profils mit negativer Ortskoordinate

$D_{max}(+X)$ = lokales Dosismaximum des X-Profils mit positiver Ortskoordinate

$D_{max}(-Y)$ = lokales Dosismaximum des Y-Profils mit negativer Ortskoordinate

$D_{max}(+Y)$ = lokales Dosismaximum des Y-Profils mit positiver Ortskoordinate

$D_{min}(MK)$ = lokales Dosisminimum beider Dosisprofile im Bereich des Messkörpers

$D_{min}(-X)$ = lokales Dosisminimum des X-Profils am Feldrand mit negativer Ortskoordinate

$D_{min}(+X)$ = lokales Dosisminimum des X-Profils am Feldrand mit positiver Ortskoordinate

$D_{min}(-Y)$ = lokales Dosisminimum des Y-Profils am Feldrand mit negativer Ortskoordinate

$D_{min}(+Y)$ = lokales Dosisminimum des Y-Profils am Feldrand mit positiver Ortskoordinate

$D_{min}(Feld)$ = einheitliches Dosisminimum beider Dosisprofile an allen Feldrändern

$X_1(MK)$ = Grenze des Messkörpers in negativer X-Richtung

$X_2(MK)$ = Grenze des Messkörpers in positiver X-Richtung

$Y_1(MK)$ = Grenze des Messkörpers in negativer Y-Richtung

$Y_2(MK)$ = Grenze des Messkörpers in positiver Y-Richtung

$X_1(Feld)$ = Lage der Feldgrenze in negativer X-Richtung

$X_2(Feld)$ = Lage der Feldgrenze in positiver X-Richtung

$Y_1(Feld)$ = Lage der Feldgrenze in negativer Y-Richtung

$Y_2(Feld)$ = Lage der Feldgrenze in positiver Y-Richtung

$\Delta X(MK)$ = Lage des Messkörpermittelpunkts in X-Richtung relativ zum EPID-Zentrum

$\Delta Y(MK)$ = Lage des Messkörpermittelpunkts in Y-Richtung relativ zum EPID-Zentrum

$\Delta CAX_x$ = Lage des Zentralstrahls des Bestrahlungsfeldes in X-Richtung relativ zum EPID-Zentrum

$\Delta CAX_Y$ = Lage des Zentralstrahls des Bestrahlungsfeldes in Y-Richtung relativ zum EPID-Zentrum

$\Delta X_{ISO}$ = Zentralstrahlabweichung relativ zum Messkörpermittelpunkt in X-Richtung (gemessen in der EPID-Ebene)

$\Delta Y_{ISO}$ = Zentralstrahlabweichung relativ zum Messkörpermittelpunkt in Y-Richtung (gemessen in der EPID-Ebene)

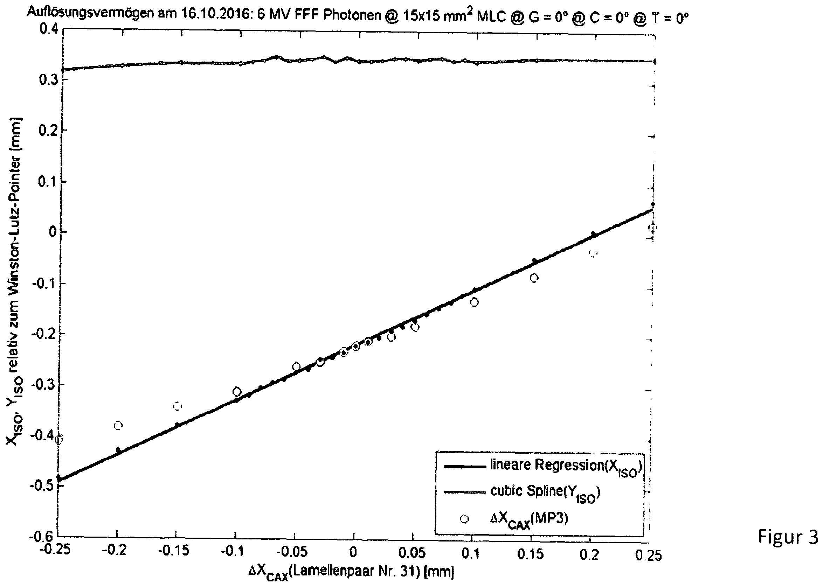

Zum Nachweis des nach dem erfindungsgemäßen Verfahren erreichbaren Ortsauflösungsvermögens von 0.01 mm werden die Lamellenpositionen eines MLC mit der kleinestmöglichen Schrittweite 0.01 mm variiert. Dabei erfolgt die Bestimmung der jeweiligen Zentralstrahllage in einem großen Wasserphantom MP3 mit einer hochauflösenden Dosimetriediode E Typ 60012 als Dosisdetektor, einem Zweikanal-Elektrometer TANDEM sowie der Software ME-PHYSTO® mc² der Firma PTW GmbH (Freiburg, Deutschland). Im Unterschied zum erfindungsgemäßen Verfahren, bei dem der Fokus-EPID-Abstand 150 cm beträgt, weist der Fokus-Detektor-Abstand zum Nachweis des Ortsauflösungsvermögens 100 cm auf. Bei der Durchführung des Nachweises des Ortsauflösungsvermögens wird eine Photonenenergie = 6 MeV, eine Dosisleistung = 400 MU/min, eine Dosisintegrationszeit je Messpunkt = 1s und eine Detektorschrittweite = 0.2 mm bis 1 mm eingestellt. In Figur 3 ist der Nachweis des Ortsauflösungsvermögens visualisiert.

Die radiologische Bestimmung der Zentralstrahlabweichungen $\Delta X_{CAX}$ beziehungsweise $\Delta Y_{CAX}$ vom theoretischen Isozentrum eines Strahlentherapiegeräts im großen Wasserphantom stellt den Goldstandard in der Strahlentherapie dar. Die schwarzen Punkte markieren die mittels der Winston-Lutz-Methode ermittelten Zentralstrahlverlagerungen zwischen einem zentral gelegenen Lamellenpaar (hier Nr. 31) in X-Richtung. Dazu wurden die das Bestrahlungsfeld begrenzenden Lamellenpaare definiert verschoben; die kleinste einstellbare Schrittweite beim verwendeten MLC "High-Definition 120" beträgt 0.01 mm. Die dazugehörige Regressionsgerade hat die Gleichung $X_{ISO}$ = 1.094 · $\Delta X_{CAX}$ - 0.217 mm. Der Korrelationskoeffizient nach Pearson beträgt r = 0.9991 bei p = 0.0000 (Wahrscheinlichkeit der Nichtkorrelation) und weist somit auf einen nahezu idealen linearen Zusammenhang hin. Die Messergebnisse im großen Wasserphantom MP3 sind als schwarze Kreise dargestellt. Mit beiden Messverfahren kann die kleinste Lamellenverschiebung $\pm 0.01$ mm aufgelöst werden. Der leicht ansteigende Verlauf der Zentralstrahlabweichung $Y_{ISO}$ während des Winston-Lutz-Tests, der hier 15 min dauerte, beweist die Relaxation des Tragarms bei 0° infolge eines auf den Tragarm und Strahlerkopf wirkenden positiven Biegemomentes um die raumfeste X-Achse. Die schwarzen Punkte der Winston-Lutz-Analyse stimmen bei $|\Delta X_{CAX}| \geq 0.05$ mm nicht mehr mit den schwarzen Kreisen der MP3-Messung überein, da im ersten Fall das Medium am Feldrand und im Feld nicht homogen ist: Luft, Kunststoff und Wolfram versus homogenes Wasser. Die Grafik in Figur 3 wurde mittels MATLAB® (Version R2007a) erzeugt.

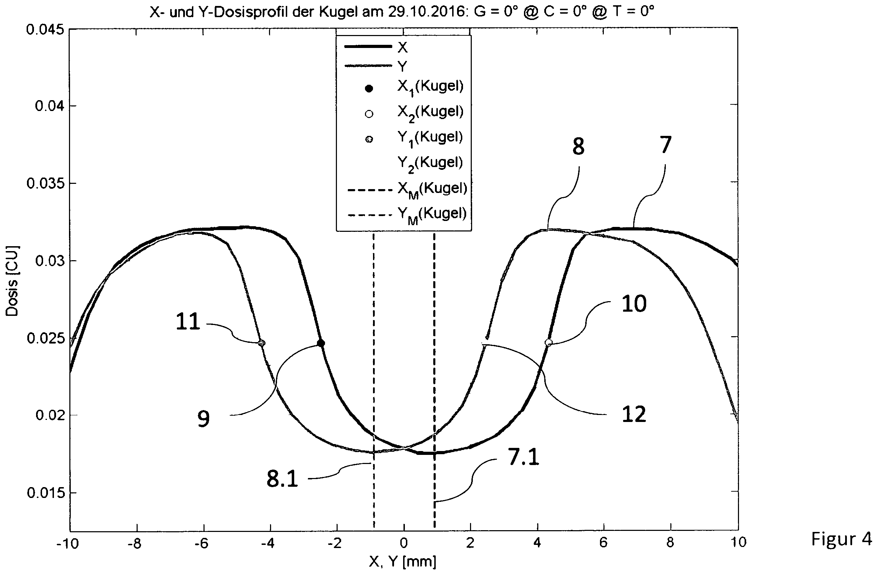

Figur 4 zeigt Dosisprofile des Winston-Lutz-Pointers als Messkörper in X-Richtung 7 und Y-Richtung 8 mit den Wendepunkten zur Definition der Messkörpergrenzen in X-Richtung 9 und 10 sowie in Y-Richtung 11 und 12. Die gestrichelten senkrechten Linien 7.1 und 8.1 kennzeichnen die Lage des Kugelmittelpunkts in X-Richtung (7.1) und in Y-Richtung (8.1). Der Streckfaktor beträgt $k^{-1}$ > 1 mit der Definition in Gleichung (1). Die Grafik in Figur 4 wurde mittels MATLAB® (Version R2007a) erzeugt.

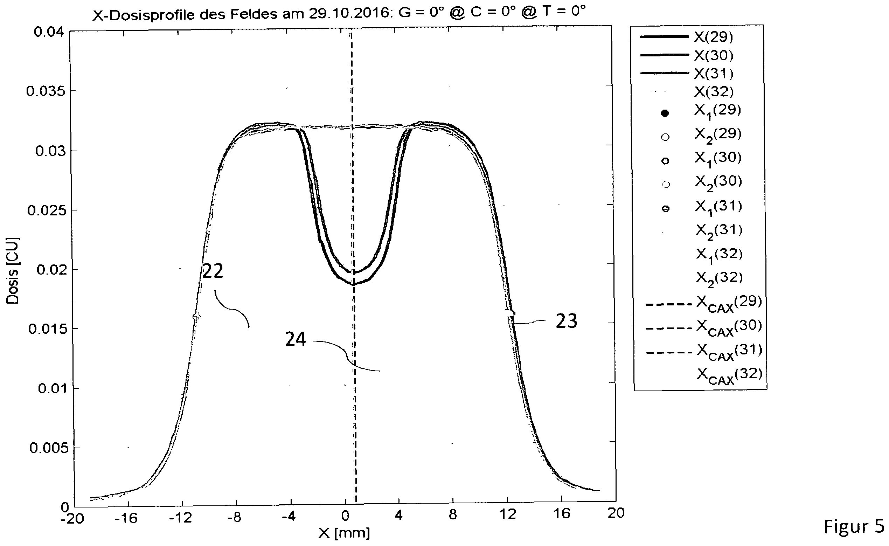

Die Figur 5 zeigt vier Dosisprofile eines MLC-begrenzten Bestrahlungsfeldes der Größe 15 × 15 mm² in X-Richtung unter den Lamellenpaaren Nr. 29 bis Nr. 32 mit den Wendepunkten 22 und 23 zur Definition der Feldgrenzen des Bestrahlungsfeldes sowie mit den Lagen der lokalen Zentralstrahlen (gestrichelte senkrechte Linien 24). Der Streckfaktor beträgt $k^{-1}$ > 1 mit der Definition in Gleichung (1). Die Grafik in Figur 5 wurde mittels MATLAB® (Version R2007a) erzeugt.

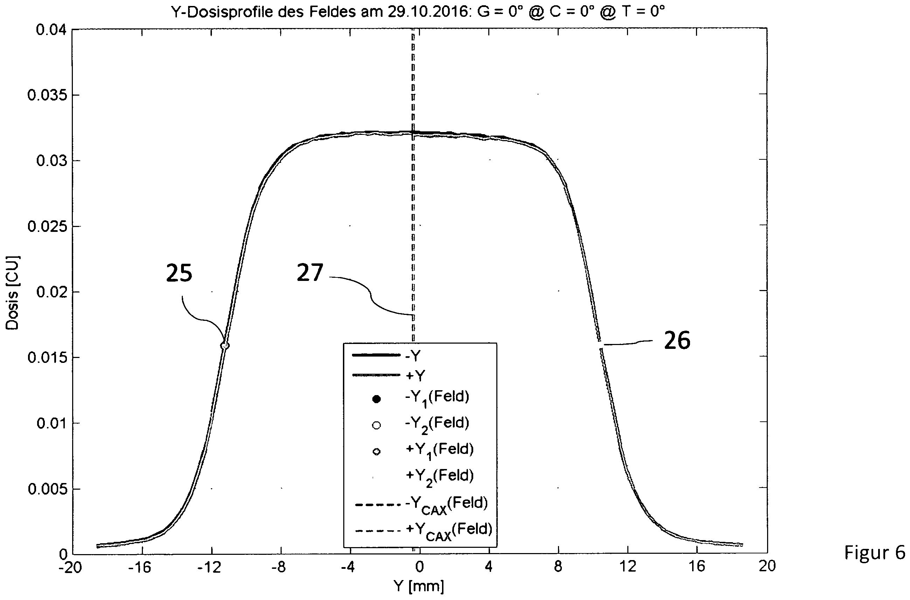

Figur 6 zeigt weitere zwei Dosisprofile eines MLC-begrenzten Bestrahlungsfeldes der Größe 15 × 15 mm² in Y-Richtung zwischen den geschlossenen Lamellenpaaren Nr. 27 und Nr. 34 mit den Wendepunkten 25 und 26 zur Definition der Feldgrenzen sowie den Lagen der lokalen Zentralstrahlen in Y-Richtung (gestrichelte senkrechte Linien 27). Der Streckfaktor beträgt $k^{-1}$ > 1 mit der Definition in Gleichung (1). Die Grafik in Figur 6 wurde mittels MATLAB® (Version R2007a) erzeugt.

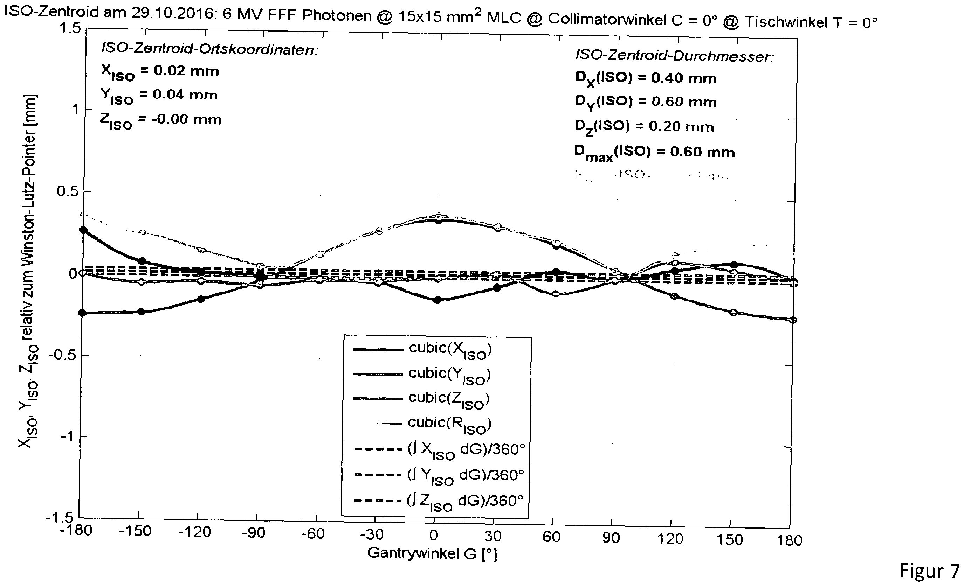

Figur 7 zeigt eine schematische Darstellung der Geometrie eines tragarmwinkelabhängigen Isozentroids eines Strahlentherapiegeräts Novalis powered by TrueBeam™ STx (VARIAN Medical Systems, Inc., Palo Alto, CA, USA und BRAINLAB AG, Feldkirchen, Deutschland). Die Volllinien in den Farben Schwarz, Dunkelgrau und Grau repräsentieren die Zentralstrahlabweichungen relativ zum Messkörper in den Richtungen X, Y beziehungsweise Z des raumfesten Koordinatensystems. Die Strichlinien markieren die Ortskoordinaten des Isozentroids in diesen Richtungen. Die Durchmesser in den Achsen des Inertialsystems, deren Maximum sowie die Ortskoordinaten werden auch als Zahlenwerte ausgegeben. Zusätzlich wird der Betrag des räumlichen Radiusvektors als Funktion des Tragarmwinkels dargestellt (hellgraue Linie) und sein Maximum angegeben. Analoge Ergebnisdarstellungen werden sowohl für die solitären collimator- und tischwinkelabhängigen Isozentroide als auch für den globalen Isozentroid, der alle drei solitären Isozentroide vereint, mittels MAT-LAB® (Version R2007a) erzeugt.

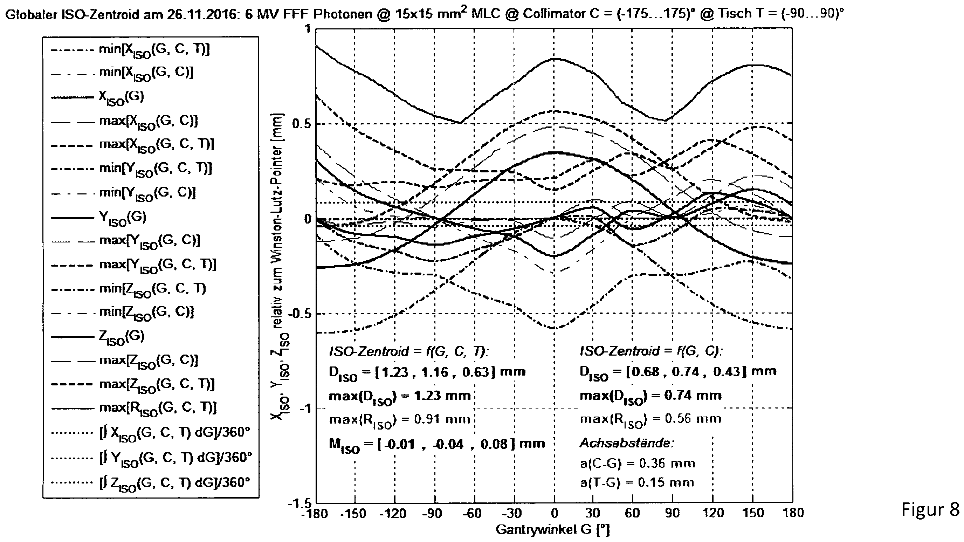

Figur 8 zeigt eine grafische Darstellung der Geometrie des globalen Isozentroids eines medizinischen Elektronen-Linearbeschleunigers Novalis powered by TrueBeam™ STx mittels MATLAB®. Die Volllinien in den Farben Schwarz, Dunkelgrau und Grau repräsentieren die tragarmwinkelabhängigen Zentralstrahlabweichungen relativ zum Winston-Lutz-Pointer in den Richtungen X, Y beziehungsweise Z des Inertialsystems. Die Grenzen der zusätzlichen negativen und positiven Streuungsbänder für die überlagerte Collimatordrehung werden als dünne Strich-Punkt-Linien beziehungsweise Strichlinien dargestellt. Bei der Hinzunahme der tischwinkelabhängigen Zentralstrahlabweichungen sind die entsprechenden Linien dick dargestellt. Die hellgraue Linie zeigt den tragarmwinkelabhängigen Verlauf des maximalen Radius des globalen Isozentroids. Die Punktlinien markieren die Ortskoordinaten des globalen Isozentroids. Die Durchmesser in den Achsen des Inertialsystems, deren Maxima sowie die Ortskoordinaten werden auch als Zahlenwerte ausgegeben. Außerdem sind im Diagramm unten rechts die räumlichen Abstände der Rotationsachsen des Collimators und Tisches relativ zur Tragarmrotationsachse ersichtlich.

## Patentansprüche

1. Verfahren zur EPID-basierten Überprüfung, Korrektur und Minimierung des Isozentrums eines Strahlentherapiegerätes, welches zumindest eines um eine Tischachse drehbaren Patientenlagerungstisch, einen um eine Tragarmachse drehbaren Tragarm, einen an dem Tragarm angeordneten Strahlerkopf zur Erzeugung eines Therapiestrahls, einen drehbaren Collimator, eine Einrichtung zum Projizieren eines radiologischen Isozentrums und ein digitales Aufnahmesystem (EPID) zum Erfassen von Dosisbildern mittels des Therapiestrahls umfasst, bei dem nach folgenden Schritten

a) ein Messkörper mittels der Projektionseinrichtung an der Projektionsposition im momentanen radiologischen Isozentrum des Strahlentherapiegeräts positioniert wird,

b) ein mit dem Collimator begrenztes Bestrahlungsfeld für mindestens eine vorgegebene Winkeleinstellung des Tragarms, des Patientenlagerungstischs und des Collimators appliziert wird, und dabei

c) mit dem EPID mindestens ein gemeinsames Dosisbild des Messkörpers und des Bestrahlungsfeldes aufgenommen wird,

d) anhand des aufgenommenen Dosisbildes jeweils ein Dosisprofil für jede Richtung innerhalb eines EPID-Koordinatensystems erstellt wird, und

e) im Verlauf des Dosisprofils an beiden zu erwartenden Körpergrenzen des Messkörpers in X-Richtung des EPID-Koordinatensystems und an beiden zu erwartenden Körpergrenzen des Messkörpers in Y- Richtung des EPID-Koordinatensystems zwischen einem lokalen Dosisminimum und einem lokalen Dosismaximum und zwischen einem lokalen Dosismaximum und einem lokalen Dosisminium jeweils ein Wendepunkt ermittelt, und

f) die ermittelten Positionen der Wendepunkte den Körpergrenzen des Messkörpers in X-Richtung und in Y-Richtung zugeordnet werden,

g) anhand der zugeordneten Körpergrenzen des Messkörpers im Dosisbild eine Lage des Mittelpunkts des Messkörpers relativ zu dem EPID-Zentrum ermittelt wird, wobei die Schritte d) bis g) analog für die Feldgrenzen und den Feldmittelpunkt des Bestrahlungsfeldes durchgeführt werden, und

h) aus einer Lageabweichung des Mittelpunktes des Messkörpers vom EPID-Zentrum und aus einer Lageabweichung des Feldmittelpunktes des Bestrahlungsfeldes vom EPID-Zentrum ein Differenzvektor bestimmt wird, und

i) die Vektorkomponenten des Differenzvektors zur Korrektur des momentanen radiologischen Isozentrums eingesetzt werden, wobei aus den einzelnen Zentralstrahlabweichungen in den drei Raumrichtungen X, Y und Z der drei räumlichen Isozentren des Tragarms, des Collimators und des Patientenlagerungstisches ein globales räumliches Isozentrum des Strahlentherapiegeräts bestimmt wird, wobei sämtliche die räumlichen Isozentren beeinflussende gerätespezifische Parameter des Strahlentherapiegeräts durch Minimierung einer vorgegebenen Zielfunktion optimiert werden.

2. Verfahren nach Anspruch 1, **dadurch gekennzeichnet, dass** die Verfahrensschritte b) bis h) für die Winkelfreiheitsgrade des Patientenlagerungstischs, des Tragarms und des Collimators mit einem Inkrement von höchstens 30° durchgeführt werden.

3. Verfahren nach einem der vorhergehenden Ansprüche 1 und 2, **dadurch gekennzeichnet, dass** aus Dosisbildern, die von verschiedenen Winkelpositionen des Patientenlagerungstischs, des Tragarms und des Collimators stammen, ermittelbare Differenzvektoren zur Bestimmung der Größe und Lage der räumlichen Isozentren eingesetzt werden, wobei die Vektorkomponenten der Ortsvektoren der räumlichen Isozentren zur Korrektur radiologischen Isozentrums eingesetzt werden.

4. Verfahren nach einem der vorhergehenden Ansprüche 1 bis 3, **dadurch gekennzeichnet, dass** das Bestrahlungsfeld unter Berücksichtigung von Mindestwerten einer Feldgröße, einer Relaxationszeit des Tragarms, einer Dosis je Bestrahlungsfeld, und/oder eines Fokus-EPID-Abstandes appliziert wird.

5. Verfahren nach einem der vorhergehenden Ansprüche 1 bis 4, **dadurch gekennzeichnet, dass** der/die Wendepunkt/e im Bereich eines 50%- Dosispunktes zwischen einem Dosisminimum und ein Dosismaximum des Dosisprofils und/oder im Bereich eines 50%-Dosispunktes zwischen einem Dosismaximum und einem Dosisminium des Dosisprofils ermittelt wird /werden.

6. Verfahren nach einem der vorhergehenden Ansprüche 1 bis 5, **dadurch gekennzeichnet, dass** der/die Wendepunkt/e im Bereich des 50%- Dosispunktes vorzugsweise zwischen zwei Pixeln festgelegt wird/werden, von denen ein erstes Pixel eine geringere Dosis als 50% und ein an das erste Pixel angrenzendes zweites Pixel eine größere Dosis als 50% repräsentiert.

7. Verfahren nach einem der vorhergehenden Ansprüche 1 bis 6, **dadurch gekennzeichnet, dass** die Ortsvektoren der räumlichen Isozentren zur Kalibrierung eines Patientenpositionierungssystems eingesetzt werden.

**8.** Verfahren nach einem der vorhergehenden Ansprüche 1 bis 7, **dadurch gekennzeichnet, dass** die Ortsvektoren der räumlichen Isozentren zur Korrektur der Projektionseinrichtung eingesetzt werden.

**9.** Verfahren nach einem der vorhergehenden Ansprüche 1 bis 8, **dadurch gekennzeichnet, dass** bei der Verwendung eines Multi-Leaf-Collimators (MLC) die Schritte d) bis h) für jedes das Bestrahlungsfeld begrenzende Lamellenpaar des MLC durchgeführt werden.

**10.** Verfahren nach einem der vorhergehenden Ansprüche 1 bis 9, **dadurch gekennzeichnet, dass** bei einer Variation des Patiententischwinkels ein Tragarmwinkel $\neq 0°$ eingestellt wird.

**11.** Verfahren nach einem der vorhergehenden Ansprüche 1 bis 10, **dadurch gekennzeichnet, dass** die Verfahrensschritte d) bis i) von einer Software gesteuert automatisch durchgeführt werden.

**12.** Verfahren nach einem der vorhergehenden Ansprüche 1 bis 11, **dadurch gekennzeichnet, dass** geometrische Toleranzen des Strahlentherapiegeräts quantifiziert und rechnerisch berücksichtigt werden.

**13.** Verfahren nach einem der vorhergehenden Ansprüche 1 bis 12, **dadurch gekennzeichnet, dass** das Isozentrum des Patientenlagerungstischs alternativ bei einem Tragarmwinkel = 0° mittels eines vom Strahlentherapiegerät unabhängigen radiologischen Patientenpositionierungssystems bestimmt wird.

**14.** Anwendung des Verfahrens nach den vorhergehenden Ansprüchen 1 bis 13 an einem Therapiesimulator, wobei die räumlichen Isozentren bestimmt, korrigiert und minimiert werden.

**Claims**

**1.** Method for the EPID-based review, correction and minimisation of the isocenter of a radiotherapy machine that comprises at least a patient support table, which is rotatable about a table axis, a supporting arm, which is rotatable about a supporting-arm axis, a radiation-emitter head, arranged on the supporting arm, for generating a therapy beam, a rotatable collimator, a device for projecting a radiological isocenter and a digital recording system (EPID) for capturing dose images by means of the therapy beam, wherein, according to the following steps:

a) by means of the projection device, a measurement element is positioned at the projection position in the current radiological isocenter of the radiotherapy machine,
b) a radiation field defined by the collimator is applied for at least one predetermined angular position of the supporting arm, of the patient support table and of the collimator, and in the process
c) at least one common dose image of the measurement element and of the radiation field is recorded by the EPID,
d) on the basis of the recorded dose image, a respective dose profile is created for each direction within an EPID coordinate system, and
e) a respective inflection point is determined over the course of the dose profile at the two expected element boundaries of the measurement element in the X-direction of the EPID coordinate system and at the two expected element boundaries of the measurement element in the Y-direction of the EPID coordinate system between a local dose minimum and a local dose maximum and between a local dose maximum and a local dose minimum, and
f) the determined positions of the inflection points are assigned to the element boundaries of the measurement element in the X-direction and in the Y-direction,
g) on the basis of the assigned element boundaries of the measurement element in the dose image, a position of the centre point of the measurement element relative to the EPID centre is determined, wherein steps d) to g) are carried out similarly for the field boundaries and the field centre point of the radiation field, and
h) a differential vector is ascertained from both a positional deviation of the centre point of the measurement element from the EPID centre and a positional deviation of the field centre point of the radiation field from the EPID centre, and
i) the vector components of the differential vector are used to correct the current radiological isocenter, wherein a global spatial isocenter of the radiotherapy machine is ascertained from the individual central-beam deviations in the three directions in space X, Y and Z of the three spatial isocenters of the supporting arm, of the collimator and of the patient support table, wherein all the machine-specific parameters of the radiotherapy machine that influence the spatial isocenters are optimised by minimising a predetermined target function.

**2.** Method according to claim 1, **characterised in that** method steps b) to h) are carried out for the degrees of angular freedom of the patient support table, of the supporting arm and of the collimator with an increment of at most 30°.

**3.** Method according to any of preceding claims 1 and 2, **characterised in that** differential vectors that can be determined from dose images, which originate from various angular positions of the patient support table, of the supporting arm and of the collimator, are used to ascertain the size and position of the spatial isocenters, wherein the vector components of the location vectors of the spatial isocenters are used to correct the radiological isocenter.

**4.** Method according to any of preceding claims 1 to 3, **characterised in that** the radiation field is applied while taking account of minimum values a of a field size, a relaxation time of the supporting arm, a dose per radiation field and/or a focus-EPID distance.

**5.** Method according to any of preceding claims 1 to 4, **characterised in that** the inflection point(s) is/are determined in the region of a 50% dose point between a dose minimum and a dose maximum of the dose profile and/or in the region of a 50% dose point between a dose maximum and a dose minimum of the dose profile.

**6.** Method according to any of preceding claims 1 to 5, **characterised in that** the inflection point(s) is/are set in the region of the 50% dose point preferably between two pixels, of which a first pixel represents a dose lower than 50% and of which a second pixel, which adjoins the first pixel, represents a dose higher than 50%.

**7.** Method according to any of preceding claims 1 to 6, **characterised in that** the location vectors of the spatial isocenters are used to calibrate a patient positioning system.

**8.** Method according to any of preceding claims 1 to 7, **characterised in that** the location vectors of the spatial isocenters are used to correct the projection device.

**9.** Method according to any of preceding claims 1 to 8, **characterised in that**, when using a multi-leaf collimator (MLC), steps d) to h) are carried out for each MLC slat pair defining the radiation field.

**10.** Method according to any of preceding claims 1 to 9, **characterised in that** a supporting-arm angle ≠ 0° is set when varying the patient table angle.

**11.** Method according to any of preceding claims 1 to 10, **characterised in that** method steps d) to i) are carried out by a piece of software in a controlled, automatic manner.

**12.** Method according to any of preceding claims 1 to 11, **characterised in that** geometric tolerances of the radiotherapy machine are quantified and taken into account in the calculations.

**13.** Method according to any of preceding claims 1 to 12, **characterised in that**, alternatively, the isocenter of the patient support table is determined at a supporting-arm angle = 0° by means of a radiological patient positioning system that is independent of the radiotherapy machine.

**14.** Use of the method according to the preceding claims 1 to 13 in a therapy simulator, wherein the spatial isocenters are ascertained, corrected and minimised.

**Revendications**

**1.** Procédé pour la surveillance, la correction et la minimisation basées sur un EPID (Electronic Portal Image Device) de l'isocentre d'un appareil de radiothérapie, lequel comprend au moins une table de support pour patients pouvant tourner autour d'un axe de table, un bras porteur pouvant tourner autour d'un axe de bras porteur, une tête d'émetteur de rayon disposée sur le bras porteur pour générer un rayon thérapeutique, un collimateur rotatif, un dispositif de projection d'un isocentre radiologique et un système d'enregistrement numérique (EPID) pour détecter des images de dose au moyen du rayon thérapeutique, où selon des étapes qui suivent :

a) un corps de mesure est positionné au moyen du dispositif de projection sur la position de projection dans l'isocentre radiologique instantané de l'appareil de radiothérapie,
b) un champ d'irradiation délimité au moyen du collimateur est appliqué pour au moins une position angulaire prédéfinie du bras porteur, de la table de support pour patients et du collimateur, et ce faisant,

c) au moins une image conjointe de dose du corps de mesure et du champ d'irradiation est enregistrée avec l'EPID,

d) respectivement un profil de dose est créé pour chaque direction au sein d'un système de coordonnées EPID à l'aide de l'image de dose enregistrée, et

e) respectivement un point de retournement est déterminé le long du profil de dose sur deux limites corporelles attendues du corps de mesure dans la direction X du système de coordonnées d'EPID et sur les deux limites corporelles attendues du corps de mesure dans la direction Y du système de coordonnées d'EPID entre un minimum de dose local et un maximum de dose local et entre un maximum de dose local et un minimum de dose local, et

f) les positions déterminées des points de retournement sont associées aux limites corporelles du corps de mesure dans la direction X et dans la direction Y,

g) une position du point central du corps de mesure par rapport au centre d'EPID est déterminée à l'aide des limites corporelles associées du corps de mesure sur l'image de dose, dans lequel les étapes d) à g) sont effectuées de manière similaire pour les limites de champ et le point central de champ du champ d'irradiation, et

h) un vecteur différentiel est défini à partir d'une déviation de position du point central du corps de mesure par rapport au centre d'EPID et à partir d'une déviation de position du point central de champ du champ d'irradiation par rapport au centre EPID, et

i) les composantes vectorielles du vecteur différentiel sont employées pour la correction de l'isocentre radiologique instantané, dans lequel un isocentre spatial global de l'appareil de radiothérapie est défini à partir des divers écarts de rayon central dans les trois directions spatiales X, Y et Z des trois isocentres spatiaux du bras porteur, du collimateur et de la table de support pour patients, dans lequel tous les paramètres spécifiques à l'appareil ayant une influence sur les isocentres spatiaux de l'appareil de radiothérapie sont optimisés par la minimisation d'une fonction ciblée prédéfinie.

2.  Procédé selon la revendication 1, **caractérisé en ce que** les étapes de procédé b) à h) sont effectuées pour les degrés de liberté d'angle de la table de support pour patients, du bras porteur et du collimateur avec un incrément de 30° au maximum.

3.  Procédé selon l'une quelconque des revendications précédentes 1 et 2, **caractérisé en ce que** des vecteurs différentiels pouvant être déterminés à partir d'images de dose, qui proviennent de différentes positions angulaires de la table de support pour patients, du bras porteur et du collimateur, sont employés pour définir la grandeur et la position des isocentres spatiaux, dans lequel les composantes vectorielles des vecteurs locaux des isocentres spatiaux sont employées pour la correction d'un isocentre radiologique.

4.  Procédé selon l'une quelconque des revendications 1 à 3, **caractérisé en ce que** le champ d'irradiation est appliqué en tenant compte de valeurs minimales d'une grandeur de champ, d'un temps de relaxation du bras porteur, d'une dose par champ d'irradiation et/ou d'un espacement focale-EPID.

5.  Procédé selon l'une quelconque des revendications précédentes 1 à 4, **caractérisé en ce que** le/les point(s) de retournement est/sont déterminé(s) dans la plage d'un point de dose à 50 % entre une dose minimale et une dose maximale du profil de dose et/ou dans la plage d'un point de dose à 50 % entre une dose maximale et une dose minimale du profil de dose.

6.  Procédé selon l'une quelconque des revendications 1 à 5, **caractérisé en ce que** le/les point(s) de retournement sont fixés dans la plage du point de dose à 50 % de préférence entre deux pixels, dont un premier pixel représente une dose inférieure à 50 % et un deuxième pixel jouxtant le premier pixel représente une dose supérieure à 50 %.

7.  Procédé selon l'une quelconque des revendications précédentes 1 à 6, **caractérisé en ce que** les vecteurs locaux des isocentres spatiaux sont employés pour l'étalonnage d'un système de positionnement de patients.

8.  Procédé selon l'une quelconque des revendications précédentes 1 à 7, **caractérisé en ce que** les vecteurs locaux des isocentres spatiaux sont employés pour la correction du dispositif de projection.

9.  Procédé selon l'une quelconque des revendications précédentes 1 à 8, **caractérisé en ce que** lors de l'utilisation d'un MLC (Multi-Leaf-Collimator), les étapes d) à h) sont effectuées pour chaque paire de lamelles, délimitant le champ d'irradiation, du MLC.

**10.** Procédé selon l'une quelconque des revendications 1 à 9, **caractérisé en ce que** dans une variation de l'angle de table pour patients, un angle de bras porteur ≠ 0° est réglé.

**11.** Procédé selon l'une quelconque des revendications 1 à 10, **caractérisé en ce que** les étapes de procédé d) à i) sont effectuées automatiquement de manière commandée par un logiciel.

**12.** Procédé selon l'une quelconque des revendications précédentes 1 à 11, **caractérisé en ce que** des tolérances géométriques de l'appareil de radiothérapie sont quantifiées et sont prises en compte sur le plan arithmétique.

**13.** Procédé selon l'une quelconque des revendications 1 à 12, **caractérisé en ce que** l'isocentre de la table de support pour patients est défini en variante pour un angle de bras porteur = 0° au moyen d'un système de positionnement de patient radiologique dépendant de l'appareil de radiothérapie.

**14.** Application du procédé selon l'une quelconque des revendications précédentes 1 à 13 sur un simulateur de thérapie, dans laquelle les isocentres spatiaux sont définis, corrigés et minimisés.

Figur 2a

X-Dosisprofil des Feldes unter Lamellenpaar Nr. 31

$a_x$ = 15.40 mm

$CAX_x$ = 0.50 mm

Legend:
- X ——
- $X_1$ ●
- $X_2$ ○
- dD/dX ——
- X[max(dD/dX)] – – –
- X[min(dD/dX)] ·······

EP 3 687 627 B1

Figur 2b

Figur 3

X- und Y-Dosisprofil der Kugel am 29.10.2016: G = 0° @ C = 0° @ T = 0°

Figur 4

Figur 5

Y-Dosisprofile des Feldes am 29.10.2016: G = 0° @ C = 0° @ T = 0°

Figur 6

ISO-Zentroid am 29.10.2016: 6 MV FFF Photonen @ 15x15 mm² MLC @ Collimatorwinkel C = 0° @ Tischwinkel T = 0°

ISO-Zentroid-Ortskoordinaten:

$X_{ISO}$ = 0.02 mm

$Y_{ISO}$ = 0.04 mm

$Z_{ISO}$ = -0.00 mm

ISO-Zentroid-Durchmesser:

$D_X(ISO)$ = 0.40 mm

$D_Y(ISO)$ = 0.60 mm

$D_Z(ISO)$ = 0.20 mm

$D_{max}(ISO)$ = 0.60 mm

cubic($X_{ISO}$)

cubic($Y_{ISO}$)

cubic($Z_{ISO}$)

cubic($R_{ISO}$)

$(\int X_{ISO}\, dG)/360°$

$(\int Y_{ISO}\, dG)/360°$

$(\int Z_{ISO}\, dG)/360°$

Gantrywinkel G [°]

$X_{ISO}, Y_{ISO}, Z_{ISO}$ relativ zum Winston-Lutz-Pointer [mm]

Figur 7

EP 3 687 627 B1

29

Figur 8

Globaler ISO-Zentroid am 26.11.2016: 6 MV FFF Photonen @ 15x15 mm² MLC @ Collimator C = (-175...175)° @ Tisch T = (-90...90)°

$X_{ISO}$, $Y_{ISO}$, $Z_{ISO}$ relativ zum Winston-Lutz-Pointer [mm]

min[$X_{ISO}$(G, C, T)]
min[$X_{ISO}$(G, C)]
$X_{ISO}$(G)
max[$X_{ISO}$(G, C)]
max[$X_{ISO}$(G, C, T)]
min[$Y_{ISO}$(G, C, T)]
min[$Y_{ISO}$(G, C)]
$Y_{ISO}$(G)
max[$Y_{ISO}$(G, C)]
max[$Y_{ISO}$(G, C, T)]
min[$Z_{ISO}$(G, C, T)]
min[$Z_{ISO}$(G, C)]
$Z_{ISO}$(G)
max[$Z_{ISO}$(G, C)]
max[$Z_{ISO}$(G, C, T)]
max[$R_{ISO}$(G, C, T)]
∫ $X_{ISO}$(G, C, T) dG]/360°
∫ $Y_{ISO}$(G, C, T) dG]/360°
∫ $Z_{ISO}$(G, C, T) dG]/360°

ISO-Zentroid = f(G, C, T):
$D_{ISO}$ = [ 1.23 , 1.16 , 0.63 ] mm
max($D_{ISO}$) = 1.23 mm
max($R_{ISO}$) = 0.91 mm
$M_{ISO}$ = [ 0.01 , -0.04 , 0.08 ] mm

ISO-Zentroid = f(G, C):
$D_{ISO}$ = [ 0.68 , 0.74 , 0.43 ] mm
max($D_{ISO}$) = 0.74 mm
max($R_{ISO}$) = 0.56 mm

Achsabstände:
a(C-G) = 0.36 mm
a(T-G) = 0.15 mm

Gantrywinkel G [°]

30

**EP 3 687 627 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **ROWSHANFARZAD, P. ; SABET, M. ; O'CONNOR, D. J. ; GREER, P. B.** Isocenter verification for linac-based stereotactic radiation therapy: review of principles and techniques. *Journal of Applied Clinical Medical Physics, Volume,* 2011, vol. 12 (4), 185-195
- **RAVINDRAN, P.B.** A study of Winston-Lutz test on two different electronic portal imaging devices and with low energy imaging. *Australasian Physical and Engineering Sciences in Medicine,* 2016, vol. 39 (3), 677-685
- **DU, W. ; YANG, J.** A robust Hough transform algorithm for determining the radiation centers of circular and rectangular fields with subpixel accuracy. *Phys. Med. Biol.,* 2009, vol. 54 (3), 555-567
- **PFEIFFER, F. ; GLOCKER, CH.** Multibody dynamics with unilateral contacts. John Wiley & Sons, Inc, 1996